(19) Europäisches Patentamt
European Patent Office
Office européen des brevets

(11) **EP 4 699 543 A1**

(12) **EUROPEAN PATENT APPLICATION**

(43) Date of publication:
**25.02.2026 Bulletin 2026/09**

(21) Application number: **25194238.9**

(22) Date of filing: **06.08.2025**

(51) International Patent Classification (IPC):
**A61B 7/04** *(2006.01)*

(52) Cooperative Patent Classification (CPC):
**A61B 7/04**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **22.08.2024 US 202463685818 P
04.08.2025 US 202519290088**

(71) Applicant: **Pacesetter, Inc.
Sylmar, CA 91342 (US)**

(72) Inventors:
- **Simonetti, Angelo
  Palma Campania (IT)**
- **Mangual-Soto, Jan O
  Rho (IT)**
- **Tsien, Li Lei
  Brussels (BE)**
- **Gill, Jong
  Valencia, CA (US)**

(74) Representative: **Barker Brettell Sweden AB
Kungsbroplan 3
112 27 Stockholm (SE)**

(54) **METHODS AND SYSTEMS FOR USING SEISMOCARDIOGRAPHY-BASED ALGORITHM FOR MONITORING HEART VALVE OPERATION**

(57)     Methods and systems (100, 500) for monitoring heart valve operation using signals (812) detected by a heart sound sensor (270, 300, 525) includes memory (260, 522) to store program instructions and one or more processors (220, 520) that, when executing the program instructions receive seismocardiograpy (SCG) signals (812) detected by a heart sound sensor (270, 300, 525) along an axis. The SCG signals (812) include heart sound signals for a series of heartbeats over a first time period. The SCG signals (812) are segmented into SCG segments (826) for corresponding heartbeats within the series of heartbeats over the first time period. A template is calculated based on a first subset of the SCG segments (826). At least a portion of a second subset of the SCG segments (826) are compared to the template to determine matching scores, and an alert is generated in response to a select number of the second subset of SCG segments (826) having the matching scores that satisfy a matching threshold.

FIG. 1

EP 4 699 543 A1

**Description**

FIELD OF THE INVENTION

Embodiments of the present disclosure generally relate to methods and systems for monitoring heart function based on heart sounds.

BACKGROUND OF THE INVENTION

Valvular heart disease (VHD) is a leading cause of cardiovascular morbidity and mortality worldwide and the resultant disease burden is projected to increase in the coming years. Such pathologies (e.g., valve stenosis, regurgitation) are commonly diagnosed via physical examination (i.e., auscultation of heart sounds) and medical imaging techniques, such as echocardiography, computed tomography, and magnetic resonance imaging. These techniques, however, are operator-dependent, expensive, time-consuming, and based on instrumentation that is not used in non-clinical settings.

According to the current guidelines, heart valve replacement is a treatment option for patients with VHDs. However, the risk for prosthetic heart valve (PHV) dysfunctions also should be considered over time. Prosthetic valve dysfunctions can be divided into the following categories: structural valve dysfunction, nonstructural valve dysfunction, endocarditis, and thrombus. Regardless of etiology, all these conditions have hemodynamic consequences and should be adequately addressed. So far, echocardiography remains the first-line technique to assess PHV performance and identify any PHV dysfunctions, while other invasive or imaging techniques could be secondarily exploited.

Currently, there is a growing interest in using wearable devices to collect diagnostic information. In the cardiovascular field, a technique called Seismocardiography (SCG) can be used to measure the vibrations produced by the beating heart using an accelerometer. For example, miniaturized accelerometers have been proposed, that utilize micro-electromechanical system (MEMS) technology, to detect heart sounds while the accelerometers are implanted within an implantable medical device (IMD).

Some fiducial points on SCG signal have been defined that are highly correlated to mechanical events over the heart cycle (e.g., valves opening and closing, atrial systole, peak atrial inflow, peak systolic outflow, early ventricular filling). Based on these fiducial points, some applications of SCG have been proposed, such as ECG-free heart rate variability assessment and pre-ejection period estimation.

A need remains for improvements that are lower-cost, less time-consuming, less operator-dependent, and/or potentially non-invasive to make a diagnosis of VHD or evaluate PHV performance over time.

SUMMARY

In accordance with embodiments herein, a system for monitoring heart valve operation comprises memory to store program instructions and one or more processors. The one or more processors, when executing the program instructions, are configured to receive seismocardiograpy (SCG) signals detected by a heart sound sensor along an axis, the SCG signals including heart sound signals for a series of heartbeats over a first time period, segment the SCG signals into SCG segments for corresponding heartbeats within the series of heartbeats over the first time period, calculate a template based on a first subset of the SCG segments, compare at least a portion of a second subset of the SCG segments to the template to determine matching scores, and generate an alert in response to a select number of the second subset of SCG segments having the matching scores that satisfy a matching threshold.

Optionally, the one or more processors are further configured to receive sensed cardiac activity (CA) signals detected by one or more electrodes over the first time period, and segment the SCG signals based on peaks detected within the CA signals.

Optionally, the CA signals comprise one or more sensed EGM signals or one or more sensed ECG signals.

Optionally, the one or more processors are further configured to calculate the template by averaging the first subset of the SCG segments.

Optionally, the one or more processors are further configured to evaluate one or more conditions based on at least one of sleep, activity, and posture, and calculate the template in response to at least one of the one or more conditions being satisfied.

Optionally, the one or more processors are further configured to increment a first counter in response to one of the SCG segments not satisfying the matching threshold.

Optionally, the one or more processors are further configured to increment a second counter in response to the first counter satisfying a first counter threshold.

Optionally, the one or more processors are further configured to, in response to receiving second SCG signals including heart sound signals for a second series of heartbeats over a second time period that is different than the first time period, calculate a second template based on a third subset of SCG segments of the second time period, compare at least a portion of a fourth subset of SCG segments of the second time period to the second template to determine matching scores, and generate an alert in response to the select number of the second subset of SCG segments and the fourth subset of SCG segments having matching scores that satisfy the matching threshold.

Optionally, the system further comprises an implantable medical device (IMD) comprising one or more electrodes configured to collect cardiac activity (CA) signals, the heart sound sensor, and a transceiver configured to wirelessly transmit at least one of the CA signals, the SCG signals, or the alert to an external device.

Optionally, the one or more processors are further configured to receive sensed cardiac activity (CA) signals detected by one or more electrodes over the first time period and analyze at least one of the CA signals and the SCG signals using artificial intelligence.

Optionally, the one or more processors are further configured to reset a counter associated with the select number in response to generating an alert.

Optionally, the matching scores are based on morphology or morphological features of the SCG signals and the template.

Optionally, monitoring the heart valve operation includes detecting valve disease and/or detecting prosthetic valve dysfunction.

Optionally, the one or more processors are further configured to filter the heart sound signals based on one or more frequency ranges or one or more axes associated with the heart sound sensor.

Optionally, the one or more processors are further configured to receive second SCG signals detected by the heart sound sensor along a second axis, the second SCG signals including heart sound signals for a series of heartbeats over a second time period, segment the second SCG signals into SCG segments for corresponding heartbeats within the series of heartbeats over the second time period, calculate a second template based on a first subset of the second SCG segments, compare at least a portion of a second subset of the second SCG segments to the second template to determine matching scores, and select the SCG signals or the second SCG signals that have the matching scores that best match the matching threshold.

Optionally, wherein the one or more processors adjust, in response to the generation of the alert, a threshold, a counter, a time period, a delay, a parameter to increase or decrease a number of alerts, pacing of an IMD, or sensing of an IMD.

In accordance with embodiments herein a method for monitoring heart valve operation comprises receiving seismocardiography (SCG) signals detected by a heart sound sensor along an axis, the SCG signals including heart sound signals for a series of heartbeats over a first time period, segmenting the SCG signals into SCG segments for corresponding heartbeats within the series of heartbeats over the first time period, calculating a template based on a first subset of the SCG segments, comparing at least a portion of a second subset of the SCG segments to the template to determine matching scores, and generating an alert in response to a select number of the second subset of SCG segments having the matching scores that satisfy a matching threshold.

Optionally, the segmenting further comprises segmenting the SCG signals based on cardiac activity (CA) signals detected over the first time period.

Optionally, the method further comprises calculating the template by averaging the first subset of the SCG segments.

Optionally, the method further comprises evaluating one or more conditions based on at least one of sleep, activity, and posture, the template being calculated in response to at least one of the one or more conditions being satisfied.

Optionally, the method further comprises filtering the heart sound signals based on one or more frequency ranges and/or one or more axes associated with the heart sound sensor.

BRIEF DESCRIPTION OF THE DRAWINGS

Figure 1 illustrates a schematic block diagram of an implantable medical device (IMD) intended for subcutaneous implantation at a site near the heart.

Figure 2 shows an example block diagram of the IMD formed in accordance with embodiments herein.

Figure 3 illustrates a schematic diagram of a heart sound (HS) sensor that may be implemented as an accelerometer in accordance with embodiments herein.

Figure 4 provides a sectional view of a patient's heart and shows a leadless IMD in accordance with embodiments herein.

Figure 5 illustrates an example block diagram of a leadless IMD in accordance with embodiments herein.

Figure 6 illustrates a computer-implemented method that discriminates between patients with valvular heart disease (VHD) and/or prosthetic heart valve (PHV) dysfunction and healthy patients in accordance with embodiments herein.

Figure 7 illustrates an example of an electrocardiogram (ECG) signal and a seismocardiogram (SCG) signal of a heartbeat that have been simultaneously acquired in accordance with embodiments herein.

Figure 8 illustrates ECG beats selected as reference for segmenting the SCG signal in accordance with embodiments herein.

Figure 9 illustrates another method that discriminates between patients with VHD and/or PHV dysfunction and healthy patients in accordance with embodiments herein.

Figure 10 illustrates examples of methods for treating a patient being monitored for VHD and/or PHV dysfunction in accordance with embodiments herein.

Figure 11 illustrates a method for obtaining SCG data over different bandwidths and axes and determining which SCG signal to use when diagnosing VHD and/or PHV dysfunction in accordance with embodiments herein.

Figure 12 shows a graph of the mean and standard deviation (Std) of morphological correlation indexes between a template (e.g., average of first 10 SCG segments) and all other SCG segments for a group of 100 pathological and 20 healthy patients in accordance with embodiments herein.

Figure 13 is a schematic illustration of a remote programming system in accordance with embodiments herein.

Figure 14 illustrates a system for verifying an update sent by a clinician from a clinician application through a remote programming engine (RPE) to an IMD of a patient that utilizes a patient application in accordance with embodiments herein.

Figure 15 illustrates a medical device remote controlled (MDRC) system capable of providing communication sessions in accordance with embodiments herein.

Figure 16 illustrates a computer-implemented method 1600 for using VHD parameters to monitor heart failure and/or other disease states in accordance with embodiments herein.

## DETAILED DESCRIPTION

It will be readily understood that the components of the embodiments as generally described and illustrated in the Figures herein, may be arranged and designed in a wide variety of different configurations in addition to the described example embodiments. Thus, the following more detailed description of the example embodiments, as represented in the Figures, is not intended to limit the scope of the embodiments, as claimed, but is merely representative of example embodiments.

Reference throughout this specification to "one embodiment" or "an embodiment" (or the like) means that a particular feature, structure, or characteristic described in connection with the embodiment is included in at least one embodiment. Thus, appearances of the phrases "in one embodiment" or "in an embodiment" or the like in various places throughout this specification are not necessarily all referring to the same embodiment.

Furthermore, the described features, structures, or characteristics may be combined in any suitable manner in one or more embodiments. In the following description, numerous specific details are provided to give a thorough understanding of embodiments. One skilled in the relevant art will recognize, however, that the various embodiments can be practiced without one or more of the specific details, or with other methods, components, materials, etc. In other instances, well-known structures, materials, or operations are not shown or described in detail to avoid obfuscation. The following description is intended only by way of example, and simply illustrates certain example embodiments.

The methods described herein may employ structures or aspects of various embodiments (e.g., systems and/or methods) discussed herein. In various embodiments, certain operations may be omitted or added, certain operations may be combined, certain operations may be performed simultaneously, certain operations may be performed concurrently, certain operations may be split into multiple operations, certain operations may be performed in a different order, or certain operations or series of operations may be re-performed in an iterative fashion. It should be noted that other methods may be used, in accordance with an embodiment herein. Further, wherein indicated, the methods may be fully or partially implemented by one or more processors of one or more devices or systems. While the operations of some methods may be described as performed by the processor(s) of one device, additionally, some or all such operations may be performed by the processor(s) of another device described herein. For example, an IMD includes IMD memory and one or more IMD processors, while each external device/system (ED) (e.g., local, remote, or anywhere within the healthcare system) includes ED memory and one or more ED processors.

Terms

The terms "posture" and "patient posture" refer to postural states and/or activity levels of a patient including supine, laying on a right side, laying on a left side, sitting, standing, isometric arm exercises (e.g., pushing, pulling, and the like), ballottement, chest thump, device pressure (e.g., top, mid, and base), arm flap, handshake, and the like.

The term "activity level" refers to intensity and/or types of activity currently experienced by a patient at a point in time, including stationary state, rest state, exercise state, walking state, and the like.

The terms "cardiac activity signal", "cardiac activity signals", "CA signal" and "CA signals" (collectively "CA signals") are used interchangeably throughout to refer to an analog or digital electrical signal recorded by two or more electrodes positioned subcutaneous or cutaneous, where the electrical signals are indicative of cardiac electrical activity. The cardiac activity may be normal/healthy or abnormal/arrhythmic. Non-limiting examples of CA signals include ECG signals collected by cutaneous electrodes, and EGM signals collected by subcutaneous electrodes and/or by electrodes positioned within or proximate to the heart wall and/or chambers of the heart. ECG and EGM are used interchangeably herein to indicate CA signals.

The term seismocardiography (SCG) refers to a recording of vibrations induced by a heartbeat. SCG signals can be detected and recorded by a heart sound detector such as an accelerometer.

The terms "health care system" and "digital health care system" are used interchangeably throughout to reference to a system that includes equipment for measuring health parameters, and communication pathways from the equipment to secondary devices. The secondary devices may be at the same location as the equipment, or remote from the equipment at a different location. The communication pathways may be wired, wireless, over the air, cellular, in the cloud, etc. In one example, the healthcare system provided may be one of the systems described in U.S. Pat. Pub. No. 2021/0020294 entitled METHODS DEVICE AND SYSTEMS FOR HOLISTIC INTEGRATED HEALTHCARE PATIENT MANAGEMENT. Other patents that describe example monitoring systems include U.S. Pat. No. 6,572,557; entitled SYSTEM AND METHOD FOR MONITORING PROGRESSION OF CARDIAC DISEASE STATE USING PHYSIOLOGIC SENSORS; U.S. Pat. No. 6,480,733 entitled METHOD FORMONITORING HEART FAILURE filed Dec. 17, 1999, to Turcott; U.S. Pat. No. 7,272,443 entitled SYSTEM AND METHOD FOR PREDICTING A HEART CONDITION BASED ON IMPEDANCE VALUES USING AN IMPLANTABLE MEDICAL DEVICE, filed Dec. 14, 2004, to Min et al; U.S. Pat. No. 7,308,309 entitled DIAGNOSING CARDIAC HEALTH UTILIZING PARAMETER TREND ANALYSIS, filed Jan. 11, 2005, to Koh; and U.S. Pat. No. 6,645,153 entitled SYSTEM AND METHOD FOR EVALUATING RISK OF MORTALITY DUE TO CONGESTIVE HEART FAILURE USING PHYSIOLOGIC SENSORS, filed Feb. 7, 2002, to Kroll et. al.

The term "obtains" and "obtaining", as used in connection with data, signals, information and the like, include at least one of i) accessing memory of an external device or remote server where the data, signals, information, etc. are stored, ii) receiving the data, signals, information, etc. over a wireless communications link between the IMD and a local external device, and/or iii) receiving the data, signals, information, etc. at a remote server over a network connection. The obtaining operation, when from the perspective of an IMD, may include sensing new signals in real time, and/or accessing memory to read stored data, signals, information, etc. from memory within the IMD. The obtaining operation, when from the perspective of a local external device, includes receiving the data, signals, information, etc. at a transceiver of the local external device where the data, signals, information, etc. are transmitted from an IMD and/or a remote server. The obtaining operation may be from the perspective of a remote server, such as when receiving the data, signals, information, etc. at a network interface from a local external device and/or directly from an IMD. The remote server may also obtain the data, signals, information, etc. from local memory and/or from other memory, such as within a cloud storage environment and/or from the memory of a workstation or clinician external programmer.

The terms "artificial intelligence", "machine learning" and "self-learning" are used interchangeably throughout and shall mean an artificial intelligence algorithm that learns from various automatic or manual inputs, such as features of interest, prior device classified arrhythmias, observations and/or data. The machine learning algorithm is adjusted over multiple iterations based on the features of interest, posture, heart sound signals, S1 COM, S2 COM, EMAT, SI, CA signals, characteristics of interest of the CA signals, prior device classified arrhythmias, observations and/or data. For example, the machine learning algorithm is adjusted by supervised learning, unsupervised learning, and/or reinforcement learning. Non-limiting examples of machine learning algorithms are a convolutional neural network, gradient boosting random forest, decision tree, K-means, deep learning, artificial neural network, and/or the like.

The term "subcutaneous" shall mean below the skin, but not intravenous. For example, a subcutaneous electrode/lead does not include an electrode/lead located in a chamber of the heart, in a vein on the heart, or in the lateral or posterior branches of the coronary sinus.

The terms "RA", "LA", "RV", and "LV" shall mean the right atrium, left atrium, right ventricle, and left ventricle, respectively.

The term "leadless" generally refers to an absence of electrically conductive leads that traverse vessels or other anatomy outside of the intra-cardiac space, while "intra-cardiac" means generally, entirely within the heart and associated vessels, such as the superior vena cava (SVC), inferior vena cava (IVC), coronary sinus (CS), coronary veins (CV), pulmonary arteries, and the like.

The term "COI" refers to a characteristic of interest within a signal. Non-limiting examples of COI from a CA signal with a PQRST complex, include an R-wave, P-wave, T-wave, and isoelectric segments. Non-limiting examples of COI from CA signals collected at an individual electrode(s) include a sensed event (e.g., an intrinsic event or evoked response). The COI may correspond to a peak of an individual sensed event, R-wave, an average or median P, R or T-wave peak and the like. In another example, the COI may include COIs of heart sound (HS) signals including S1 amplitudes, S2 amplitudes, S3 amplitudes, S4 amplitudes, S1 to S2 intervals, S2 to S3 intervals, S3 to S4 intervals, S4 to S1 intervals, or the like.

The terms "processor," "a processor", "one or more processors" and "the processor" shall mean one or more processors. The one or more processors may be implemented by one, or by a combination of more than one implantable medical device, a wearable device, a local device, a remote device, a server computing device, a network of server computing devices and the like. The one or more processors may be implemented at a common location or at distributed locations. The one or more processors may implement the various operations described herein in a serial or parallel manner, in a shared-resource configuration and the like.

Overview

In accordance with new and unique aspects herein, methods, devices, and systems are described that utilize signals acquired by a heart sound sensor, such as an accelerometer, to monitor heart valve operation, including both valvular heart disease (VHD) and prosthetic heart valve (PHV) dysfunction diagnosis. Miniaturized accelerometers using micro-electro-mechanical-system (MEMS) technology have made HS detection possible from implantable medical devices (IMD). The same accelerometer can be utilized to detect movement of the patient during day-to-day activities, the posture based on the position and/or orientation of the IMD, and the migration of the device. For example, an IMD, such as an implantable cardiac monitor (ICM), pacemaker, etc., can incorporate a three-dimensional (3-D) accelerometer to simultaneously record heart sounds (HS) together with electrocardiogram (ECG). In other cases, the accelerometer can be implanted separately within a patient or within an external monitor applied to a patient's chest.

The accelerometer acquires seismocardiography (SCG) signals for a series of heartbeats over a time period. The SCG signals can be filtered, such as by frequency and/or axis of the accelerometer (e.g., 7.5 Hz - 100 Hz or 15 Hz - 100 Hz and any of the x/y/z axes), and/or based on breathing cycle. In some embodiments an ECG or IEGM signal is used to temporalize and segmentize the SCG signal into SCG segments for corresponding heartbeats. The beat-by-beat variability of the SCG segments is used to identify a pathologic condition associated with heart valve and/or prosthetic heart valve that has a higher variability than a non-pathologic condition.

The variability could be measured periodically (e.g., every N hours or number of heartbeats, daily, weekly, monthly), leveraging the morphology of the entire signal without delving into the extraction of specific morphological features. In other embodiments, morphological features such as the difference of peak-to-peak amplitudes between the smallest SCG peak and the largest SCG peak, the standard deviation of the width of the SCG signal, intervals between R-wave peak to one or more SCG peaks, and others can be considered. This disclosure describes different methods and systems to detect VHD and PHV dysfunctions based on the SCG signal and generate alerts and/or treatment for the patient experiencing a pathologic condition.

While in some cases the accelerometer may acquire data along one axis, in accordance with new and unique aspects herein an IMD can incorporate a 3-D accelerometer capable of detecting HS in more than one axis, such as 3 orthogonal (X, Y and Z) axes. The SCG signals can be detected in two or more axes, and each axis can be filtered based on a frequency range to identify the axis, frequency range, and/or axis/frequency range combination wherein the SCG segments have a lower beat-by-beat variability.

In accordance with new and unique aspects, a technical advantage of identifying VHD and PHV dysfunction is provided. The SCG and ECG/IEGM data can be acquired with little or no action required of the patient, and can be acquired anywhere outside or inside of a medical facility without the assistance of medical personnel. The patient can receive timely intervention and treatment as alerts are generated when possible VHD or PHV dysfunction is detected. Settings of the IMD can be automatically adjusted and/or treatments can be deployed (e.g., pacing, sensing, medication) based on the alerts. Treatments can also be adjusted/implemented remotely without action by the patient.

Implantable Medical Device

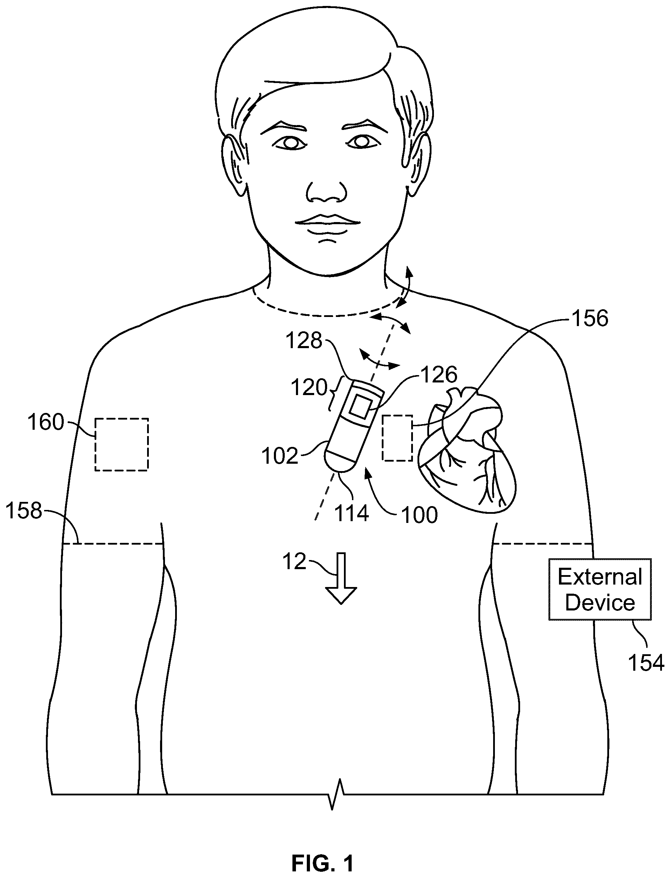

Figure 1 illustrates an IMD 100 intended for subcutaneous implantation at a site near the heart in accordance with embodiments herein. For example, the IMD and related sensors, devices, systems, etc., as described herein can be at least one of the IMDs, sensors, devices, systems, etc., illustrated and described in U.S. Pub. No. 2022/0361837, filed February 8, 2022, entitled METHOD AND SYSTEM FOR MONITORING HEART FUNCTION BASED ON HEART SOUND CENTER OF MASS; U.S. Pub. No. 2022/0361818, filed February 8, 2022, entitled METHOD AND SYSTEM FOR MONITORING HEART FUNCTION BASED ON HEART SOUND CENTER OF MASS; and U.S. Pub. No. 2024/0424307, filed June 14, 2024, entitled METHOD AND SYSTEM FOR MONITORING HEART FUNCTION BASED ON ELECTROMECHANICAL ACTIVATION TIME.

The IMD 100 includes a pair of spaced-apart sense electrodes 114, 126 positioned with respect to a housing 102. The sense electrodes 114, 126 provide for detection of far field electrogram signals. Numerous configurations of electrode arrangements are possible. For example, the electrode 114 may be located on a distal end of the IMD 100, while the electrode 126 is located on a proximal side of the IMD 100. Additionally or alternatively, electrodes 126 may be located on opposite sides of the IMD 100, opposite ends or elsewhere. The distal electrode 114 may be formed as part of the housing 102, for example, by coating all but a portion of the housing with a nonconductive material such that the uncoated portion forms the electrode 114. In this case, the electrode 126 may be electrically isolated from the housing 102 electrode by placing it on a component separate from the housing 102, such as the header 120. Optionally, the header 120 may be formed as an integral portion of the housing 102. The header 120 includes an antenna 128 and the electrode 126. The antenna 128 is configured to wirelessly communicate with an external device 154 in accordance with one or more predetermined wireless protocols (e.g., Bluetooth, Bluetooth low energy, Wi-Fi, etc.). In one example, the external device 154 is a proximate external device located proximate to or adjacent to a patient. An external device located in the same room as the patient is a proximate external device. Alternatively, the external device can be a remote external device where the external device is located a long distance from the patient such as at least one-hundred meters, in a different room, in a different building, at least five miles away, at least ten miles away, at least one-hundred miles away, etc.

The housing 102 includes various other components such as: sense electronics for receiving signals from the electrodes, a microprocessor for analyzing the far field CA signals, including assessing the presence of R-waves in cardiac beats occurring while the IMD is in different IMD locations relative to gravitational force, a loop memory for temporary storage of CA data, a device memory for long-term storage of CA data, sensors for detecting patient activity, including an accelerometer for detecting acceleration signatures (e.g., SCG signals) indicative of heart sound, and a battery for powering components.

In at least some embodiments, the IMD 100 is configured to be placed subcutaneously utilizing a minimally invasive approach. Subcutaneous electrodes are provided on the housing 102 to simplify the implant procedure and eliminate a need for a transvenous lead system. The sensing electrodes may be located on opposite sides of the device and designed to provide robust episode detection through consistent contact at a sensor-tissue interface. The IMD 100 may be configured to be activated by the patient or automatically activated, in connection with recording subcutaneous ECG signals.

The IMD 100 senses far field, subcutaneous CA signals, processes the CA signals to detect arrhythmias and if an arrhythmia is detected, automatically records the CA signals in memory for subsequent transmission to an external device 154.

The IMD 100 is implanted in a position and orientation such that, when the patient stands, the IMD 100 is located at a reference position and orientation with respect to a global coordinate system 10 that is defined relative to a gravitational direction 12. For example, the gravitational direction 12 is along the Z-axis while the X-axis is between the left and right arms.

As explained herein, the IMD 100 includes electrodes that collect cardiac activity (CA) signals in connection with multiple cardiac beats and in connection with different IMD locations (e.g., different positions and/or different orientations). The IMD 100 also includes one or more sensors to collect acceleration signatures that are indicative of heart sounds produced at different points in a cardiac cycle.

In other embodiments, an external monitor 156 can include a heart sound sensor such as an accelerometer or gyroscope. The external monitor 156 can sense SCG signals and ECG signals. The external monitor can be an external cardiac monitor such as a Holter monitor or a handheld device such as a smartphone. In some cases, external leads (not shown) can be used to acquire the ECG signals. The external monitor can be affixed to a patient's torso, such as with adhesive, or held in place by the patient when the patient is lying prone.

In still further embodiments, an external monitor 156 can be integrated into a garment 158, such as a compression shirt. The garment 158 serves to hold components of the integrated monitor in contact with the patient's torso. It should be understood that external ECG signals can be detected by other monitors (e.g., smartwatch) located on other portions of the patient's body.

Each of the external monitors 156 can communicate with the external device 154 locally or remotely.

A medication delivery device 160 is also shown and can communicate with the external device 154. The medication delivery device 160 can be implantable or affixed to the skin of the patient and is capable of dynamically delivering a controlled amount of medicine. In some embodiments, based on the analysis of the SCG signals, the external device 154 can direct the medication delivery device 160 to increase, decrease, start, and/or stop delivering one or more medicines, such as a blood thinner.

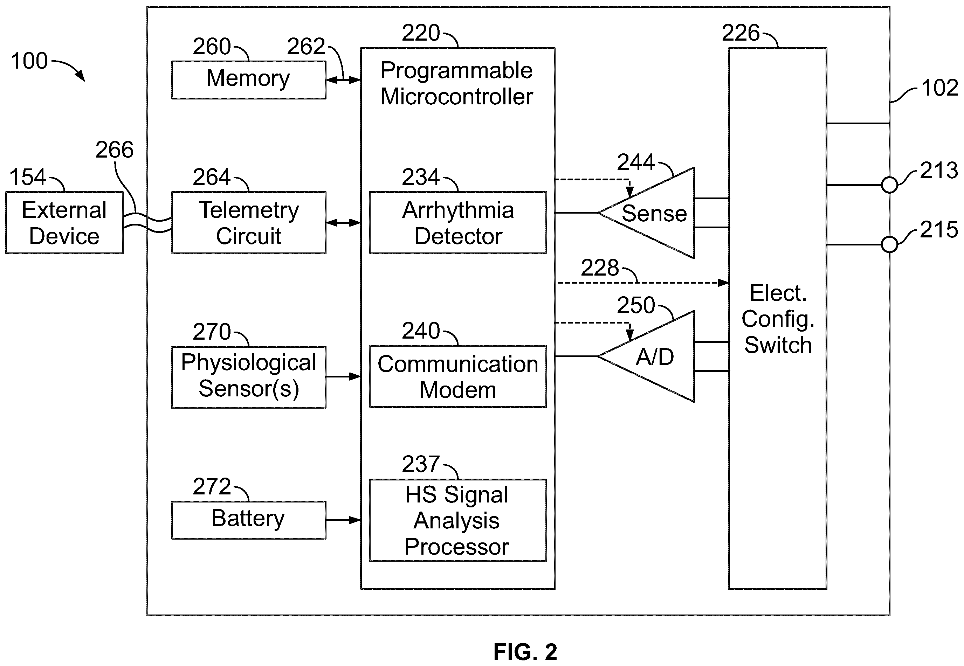

Figure 2 shows an example block diagram of the IMD 100 formed in accordance with embodiments herein. The IMD 100 may be implemented to monitor ventricular activity alone, or both ventricular and atrial activity through sensing circuit. The IMD 100 has a housing 102 to hold the electronic/computing components. The housing 102 (which is often referred to as the "can," "case," "encasing," or "case electrode") may be programmably selected to act as an electrode for certain sensing modes. Housing 102 further includes a connector (not shown) with at least one terminal 213 and optionally additional terminals 215. The terminals 213, 215 may be coupled to sensing electrodes that are provided upon or immediately adjacent the housing 102. Optionally, more than two terminals 213, 215 may be provided to support more than two sensing electrodes, such as for a bipolar sensing scheme that uses the housing 102 as a reference electrode. Additionally or alternatively, the terminals 213, 215 may be connected to one or more leads having one or more electrodes provided thereon, where the electrodes are located in various locations about the heart. The type and location of each electrode may vary.

The IMD 100 includes a programmable microcontroller 220 that controls various operations of the IMD 100, including cardiac monitoring. Microcontroller 220 includes a microprocessor (or equivalent control circuitry), RAM and/or ROM memory, logic and timing circuitry, state machine circuitry, and I/O circuitry. Microcontroller 220 includes an arrhythmia detector 234 that is configured to analyze the far field cardiac activity signals to identify the existence of an arrhythmia. The arrhythmia detector 234 analyzes the CA signals to assess a presence or absence of R-waves within the cardiac beats from a first segment of the CA signals and detect an arrythmia based on the presence or absence of one or more R-waves from the cardiac beats within a second segment of the CA signals.

The microcontroller 220 also includes an HS signal analysis (HSA) processor 237 for monitoring heart valve operation. The HSA processor 237 is configured to implement one or more of the operations discussed herein to monitor heart valve operation, which can include detecting valve disease and/or detecting prosthetic valve dysfunction. The HSA processor 237 receives SCG signals detected by a heart sound sensor 270, which can be a 3D accelerometer. The heart sound sensor 270 is also referred to herein as a physiological sensor. The SCG signals include heart sound signals for a series of heartbeats over a first time period. The HSA processor 237 segments the SCG signals into SCG segments for corresponding heartbeats within the series of heartbeats over the first time period, and calculates a template based on a first subset of the SCG segments. The HSA processor 237 compares at least a portion of a second subset of the SCG segments to the template to determine matching scores, and can generate an alert in response to a select number of the second subset of SCG segments having the matching scores that satisfy a matching threshold. In some cases, the HSA processor 237 can reset a counter associated with the select number in response to generating an alert.

In some embodiments, the matching scores are based on morphology or morphological features of the SCG signals and the template. In other cases, the HSA processor 237 can increment a first counter in response to one of the SCG segments not satisfying the matching threshold, and can increment a second counter in response to the first counter satisfying a first counter threshold.

In some embodiments, the HSA processor 237, in response to receiving second SCG signals including heart sound signals for a second series of heartbeats over a second time period that is different than the first time period, can calculate a second template based on a third subset of SCG segments of the second time period, compare at least a portion of a fourth subset of SCG segments of the second time period to the second template to determine matching scores, and generate an alert in response to the select number of the second subset of SCG segments and the fourth subset of SCG segments having matching scores that satisfy the matching threshold.

The microcontroller 220 manages storage, such as within memory 260, of alerts, counters and thresholds associated with using the SCG signals to monitor heart valve operation. The external device 154 is configured to wirelessly communicate with the IMD 100. The external device 154 includes external device (ED) memory and one or more ED processors. The one or more ED processors may be configured to perform at least one of the segment, calculate, compare and generate operations. As one example, the external device 154 may wirelessly receive the SCG and EGM signals, and the one or more ED processors may perform the segment, calculate, compare and generate operations. Further, the one or more ED processors can be configured to perform any of the operations of the HSA processor 237 to monitor heart valve operation.

In another example, the HSA processor 237 obtains the HS data collected from a physiologic sensor 270 that is a 3D accelerometer related to heart sounds (e.g., S1, S2, and S3 sounds) that represent various parts of the cardiac cycle. In some embodiments, 3D accelerometer supports the following modes: standby, single axis (1D activity), normal (3D activity/posture), and HS. Each mode is exclusive (only one mode can occur at a time), hence HS data collection cannot occur in parallel with 3D activity and posture. In HS mode, the 3D accelerometer can detect HS in three axes (X, Y, Z) and in two HS bandpass filter settings: wideband (WB), (e.g., with a frequency range of 7.5 - 100 Hz), and narrowband (NB), (e.g., with a frequency range of 15 - 100 Hz), for a total of six bandwidth HS components. The HS sensor can be configured to output one bandwidth HS component at a time; hence, HS data can be collected sequentially for each of the six bandwidth HS components rather than collecting data for all six bandwidth HS components at once. In other embodiments, a 3D accelerometer may be capable of acquiring HS data in more than one axis simultaneously.

The IMD 100 may further include one or more physiologic sensors 270. For example, the physiologic sensor 270 can be a HS sensor and may represent one or more accelerometers, such as a three-dimensional (3D) accelerometer, and/or one or more gyroscopes. The sensor 270 may utilize a piezoelectric, a piezoresistive, and/or capacitive components commonly used to convert the mechanical motion of the 3D accelerometer into an electrical signal received by the microcontroller 220. By way of example, the 3-D accelerometer may generate three electrical signals indicative of motion in three corresponding directions, namely X, Y and Z directions. The electrical signals associated with each of the three directional components may be divided into different frequency components to obtain different types of information therefrom.

In one example the 3D accelerometer can include three DC sensors that can be considered superior-inferior (X-axis), anterior-posterior (Y-axis), and lateral-medial (Z-axis), respectively. Each DC sensor can also generate the activity level of the patient, i.e., AC acceleration. Each of the DC sensors is preferably a surface micromachined integrated circuit with signal conditioning as is well known in the art. Employing surface micromachining, a set of movable capacitor plates are formed extending in a pattern from a shaped polysilicon proof mass suspended by tethers with respect to a further set of fixed polysilicon capacitor plates. The proof mass has a sensitive axis along which a force between 0 G and +50 G effects the physical movement of the proof mass and a change in the measured capacitance between the fixed and moveable plates. The measured capacitance is transformed by the on-chip signal conditioning circuits into a low voltage signal. However, many other types of accelerometers are commercially available, and it would be obvious to one of ordinary skill in the art to use other types of accelerometers in place of the above-described multi-axis DC accelerometer. While in one example three or more DC sensors are utilized, in other examples systems can be formed from two DC sensors.

To determine both the activity level and the body position of the patient, a controller can monitor the output of each of the DC sensors. Preferably using standard analog to digital conversion techniques, the output of each of the DC sensors is filtered to separate an AC signal component (representing the activity level) and a DC signal component (representing the body position). Then, the two resultant signals are further processed to determine two corresponding digital outputs which represent the instantaneous signal level of each signal. The resulting activity digital signals are then further processed to determine the activity level of the patient by methods well known within the art.

In another example, the physiologic sensor 270 collects device location information with respect to gravitational force while the IMD 100 collects CA signals in connection with multiple cardiac beats. While shown as being included within the housing 102, the physiologic sensor(s) 270 may be external to the housing 102, yet still, be implanted within or carried by the patient.

Although not shown, the microcontroller 220 may further include other dedicated circuitry and/or firmware/software components that assist in monitoring various conditions of the patient's heart and managing pacing therapies. A switch 226 is optionally provided to allow selection of different electrode configurations under the control of the microcontroller 220. The switch 226 is controlled by a control signal 228 from the microcontroller 220. The IMD 100 may be further equipped with a communication modem (modulator/demodulator) 240 to enable wireless communication. In one implementation, the communication modem 240 uses high frequency modulation, for example using RF, Bluetooth or Bluetooth Low Energy telemetry protocols. The signals are transmitted in a high frequency range and will travel through the body tissue in fluids without stimulating the heart or being felt by the patient. The communication modem 240 may be implemented in hardware as part of the microcontroller 220, or as software/firmware instructions programmed into and executed by the microcontroller 220. Alternatively, the modem 240 may reside separately from the microcontroller as a standalone component. The modem 240 facilitates data retrieval from a remote monitoring network. The modem 240 enables timely and accurate data transfer directly from the patient to an electronic device utilized by a physician.

The IMD 100 includes sensing circuit 244 selectively coupled to one or more electrodes that perform sensing operations through the switch 226 to detect CA data indicative of cardiac activity. The sensing circuit 244 may include dedicated sense amplifiers, multiplexed amplifiers, or shared amplifiers. It may further employ one or more low power, precision amplifiers with programmable gain and/or automatic gain control, bandpass filtering, and threshold detection circuit to selectively sense the features of interest. In one embodiment, switch 226 may be used to determine the sensing polarity of the CA signal by selectively closing the appropriate switches. The IMD 100 further includes an analog-to-digital A/D data acquisition system (DAS) 250 coupled to one or more electrodes via the switch 226 to sample CA signals across any pair of desired electrodes. The HSA processor 237 may be applied to signals from the sensing circuit 244 and/or the DAS 250.

By way of example, the external device 154 may represent a bedside monitor installed in a patient's home and utilized to communicate with the IMD 100 while the patient is at home, in bed and/or asleep. The external device 154 may be a programmer used in the clinic to interrogate the IMD 100, retrieve data and program detection criteria and other features. The external device 154 may be a handheld device (e.g., smartphone, tablet device, laptop computer, smartwatch, and the like) that may be coupled over a network (e.g., the Internet) to a remote monitoring service, medical network, and the like. The external device 154 may communicate with a telemetry circuit 264 (e.g., transceiver) of the IMD through a communication link 266. The external device 154 facilitates access by physicians to patient data as well as permitting the physician to review real-time and/or recorded CA signals and SCG signals collected by the IMD 100.

The microcontroller 220 is coupled to a memory 260 by a suitable data/address bus 262. The memory 260 stores the motion data, baseline motion data sets, CA signals, SCG signals, characteristics of interest associated with the CA signals and/or SCG signals, alert data, thresholds, counters, as well as the markers and other data content associated with detection and determination of the arrhythmia.

A battery 272 provides operating power to all of the components in the IMD 100. The battery 272 is capable of operating at low current drains for long periods of time. The battery 272 also desirably has a predictable discharge characteristic so that elective replacement time can be detected.

The HSA processor 237 receives the sensed cardiac activity (CA) signals detected by one or more electrodes over the first time period, wherein the segmentation of the SCG signals is based on peaks detected within the CA signals. In some cases, the CA signals can be one or more sensed EGM signals or one or more sensed ECG signals. The HSA processor 237 can calculate the template by averaging the first subset of the SCG segments.

In some cases, the HSA processor 237 can evaluate one or more conditions based on at least one of sleep, activity, and posture, and calculate the template in response to at least one of the one or more conditions being satisfied.

In some embodiments, the HSA processor 237 can filter the heart sound signals based on one or more frequency ranges or one or more axes associated with the heart sound sensor.

In other embodiments, the HSA processor 237 can receive sensed cardiac activity (CA) signals detected by one or more electrodes over a first time period, and analyze at least one of the CA signals and the SCG signals using artificial intelligence.

In other embodiments, the HSA processor 237 can receive second SCG signals detected by the heart sound sensor along a second axis, the second SCG signals including heart sound signals for a series of heartbeats over a second time period, segment the second SCG signals into SCG segments for corresponding heartbeats within the series of heartbeats over the second time period, calculate a second template based on a first subset of the second SCG segments, compare at least a portion of a second subset of the second SCG segments to the second template to determine matching scores, and select the SCG signals or the second SCG signals that have the matching scores that best match the matching threshold.

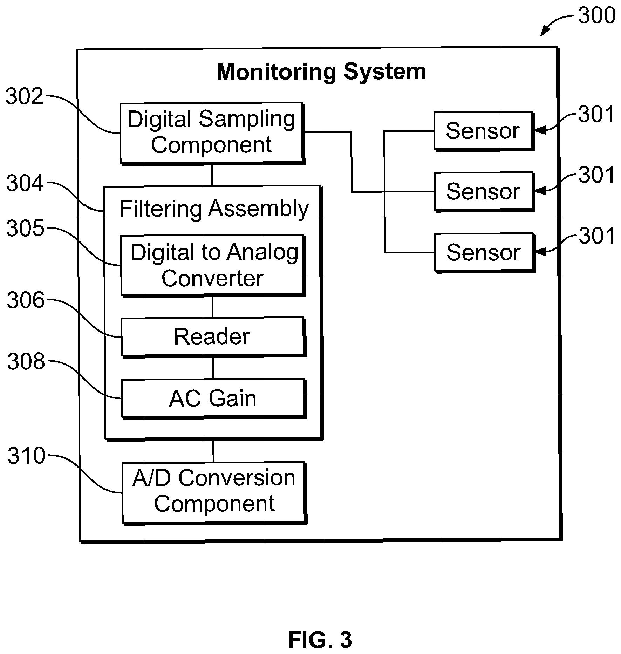

Figure 3 illustrates a schematic diagram of a HS sensor (e.g., such as physiologic sensor 270) that may be implemented as an accelerometer, more generally referred to herein as a monitoring system 300. The monitoring system 300 is used to detect and determine heart sound signals. In one embodiment, the monitoring system 300 is a three-dimensional accelerometer that may be implemented as a chip for placement in an IMD. In another embodiment, the accelerometer is formed and operates in the manner described in US patent 6,937,900, titled AC/DC MULTI-AXIS ACCELEROMETER FOR DETERMINING A PATIENT ACTIVITY AND BODY POSITION. In yet another embodiment, the accelerometer is formed and operates in the manner described in US Patent Publication 2021/0350931, titled METHOD AND SYSTEM FOR HEART CONDITION DETECTION USING AN ACCELEROMETER.

The accelerometer can include sensors that generate first (X), second (Y) and third (Z) accelerometer signals along corresponding X, Y and Z axes (also referred to as first axis accelerometer or HS signals, second axis accelerometer or HS signals and third axis accelerometer or HS signals). The X, Y and Z accelerometer signals collectively define a three-dimensional, or multi-dimensional (MD) accelerometer or HS data set. While examples herein are described in connection with an accelerometer that generates accelerometer signals along three orthogonal axes, it is recognized that embodiments may be implemented wherein accelerometer signals are generated along two or more axes, including more than three axes.

Each of the first (X), second (Y) and third (Z) accelerometer signals can further be divided into at least first and second bandwidth frequency ranges. The first and second bandwidth frequency ranges differ from one another. For example, the first and second bandwidth frequency ranges may be mutually exclusive of one another (e.g., a lower frequency range that does not overlap a higher frequency range). Alternatively, the first and second frequency ranges may partially overlap (e.g., a lower frequency range that has an upper portion that partially overlaps a lower portion of a higher frequency range). Alternatively, the first frequency range may be a subset of the second frequency range. As an example, accelerometer signals can be obtained on each axis at two HS bandpass filter settings: wideband (WB), (e.g., with a frequency range of 7.5 - 100 Hz), and narrowband (NB), (e.g., with a frequency range of 15 - 100 Hz, for a total of six bandwidth HS components). A first bandwidth HS component (first/X axis within a first frequency/NB), a second bandwidth HS component (first /X axis within a second frequency/WB), a third bandwidth HS component (second/Y axis within a first frequency/NB), a fourth bandwidth HS component (second/Y axis within a second frequency/WB), a fifth bandwidth HS component (third/Z axis within a first frequency/NB), or a sixth bandwidth HS component (third/Z axis within a second frequency/WB) may be obtained from the accelerometer.

The monitoring system 300, or HS sensor, may include a plurality of sensors 301 that monitor and receive signals from the X, Y and Z axes to obtain accelerometer data. In one embodiment, the individual X, Y and Z signals are received by a digital sampling component 302 that receives a digital input. Coupled to the digital sampling component 302 is a filtering assembly 304 that may include a digital to analog converter 305 to form an alternating current (AC) signal, a reader device 306, and an AC gain device 308. While in this embodiment, the filtering assembly includes the devices provided, in other examples, other devices may be utilized to filter the digital input signal for processing.

The monitoring system 300 may also include an analog to digital conversion component 310, along with a position, or direct current (DC) component. In one example, the analog to digital conversion component may be a 3-bit analog to digital converter (ADC). The evaluation version of the monitoring system 300 may provide 3-axis (X and Y along the chip, Z normal to the chip) DC-coupled posture signal corresponding to 3 orthogonal directions as well as 3-axis AC-coupled activity signal. In one embodiment, each of the 6 signals (and corresponding bandwidth HS components) may be sampled at 100 Hz and accumulated over 1 sec for a total of 12 signals([X/Y/Z], [posture/activity], [100/1 Hz]). This accelerometer data may be used to describe embodiments herein.

While described as a digital signal in relation to Figure 3, in other embodiments the signal may be an analog signal, filtered, amplified, etc. The accelerometer data signals may be recorded in a data storage of the accelerometer, of an IMD, of a remote device etc. Alternatively, the accelerometer data set may be obtained from a remote device or received from a storage device coupled to the accelerometer. In one example, the accelerometer data set may be a multi-dimensional accelerometer data set.

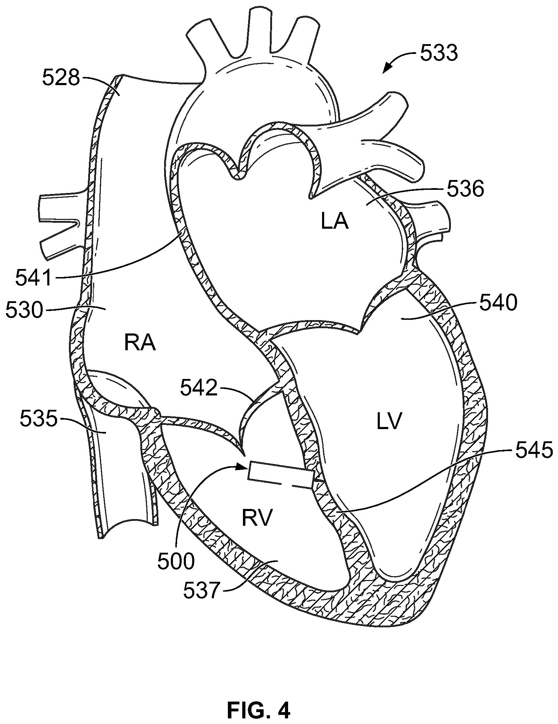

Figure 4 provides a sectional view of a patient's heart 533 and shows a leadless implantable medical device (IMD) 500. The IMD 500 has been placed through the superior vena cava 528 into the right atrium 530 of the heart 533. Figure 4 also shows the inferior vena cava 535, the left atrium 536, the right ventricle 537, the left ventricle 540, the atrial septum 541 that divides the two atria 530, 536, and the tricuspid valve 542 between the right atrium 530 and the right ventricle 537.

The IMD 500 is formed in accordance with an embodiment. The IMD 500 may represent a pacemaker, a cardiac resynchronization therapy (CRT) device, a cardioverter, a cardiac rhythm management (CRM) device, a defibrillator, or the like. The IMD 500 comprises a housing 502 configured to be implanted entirely within a single local chamber of the heart 533, such as entirely and solely within the right atrium 530, left atrium 536, the right ventricle 537 or the left ventricle 540, for example. Optionally, the IMD 500 may be implanted outside of the chambers of the heart but located in a vessel proximate to, or attached to an exterior wall of, the heart proximate to the RA, LA, RV or LV.

The chamber in which the IMD 500 is implanted in (or closest proximally to) is referred to as the "local" chamber. The local chamber includes a local chamber wall that is physiologically responsive to local activation events originating in the local chamber. The local chamber is at least partially surrounded by local wall tissue that forms, contains, or constitutes at least part of a conduction network for the associated chamber.

As shown in Figure 4, the local chamber in which the IMD 500 is implanted is the right ventricle 537. For example, the IMD 500 is mounted or fixated to the tissue wall of the right ventricle 537 along the septum 545 that divides the right ventricle 537 from the left ventricle 540. The septum 545 wall tissue in the right ventricle 537 may behave physiologically differently than the non-septum ventricular wall tissue. Optionally, the IMD 500 may be implanted in other regions of the RV, in other chambers of the heart, in vessels along an exterior of a local chamber or implanted in the exterior wall of the heart proximate to a local chamber (e.g., through the epicardium and into the myocardium). Figure 4 shows the IMD 500 in the septal wall of the RV, but optionally, the IMD 500 may be implanted at a higher location proximate to the HIS bundle in the RV. Optionally, the IMD 500 may be implanted in the RA or elsewhere. Alternatively, multiple IMDs may be implanted into the patient's heart 533 within different chambers or different segments of the same chamber.

The leadless IMD 500 may sense various intrinsic events and deliver corresponding therapies depending upon whether a subsequent intrinsic event is detected within a certain time period. For example, the IMD may utilize one or more atrial-ventricular (AV) delays to manage ventricular pacing in the event an intrinsic ventricular event does not occur within a programmed time period following a preceding intrinsic (or paced) atrial event. Similarly, the IMD may utilize one or more ventricular-ventricular (VV) delays to manage synchronization between right and left side ventricular activity. For example, a leadless IMD in the RV may deliver a paced event when an intrinsic right side ventricular event does not occur within a programmed time period following a preceding intrinsic (or paced) ventricular event in the left ventricular chamber, or vice versa. As another example, the IMD may utilize one or more atrial-HIS (AH) delays to manage HIS bundle pacing in the event an intrinsic event does not occur at the HIS bundle within a programmed time period following a preceding intrinsic (or paced) atrial event. As another example, when the IMD is implanted in the atrium, the IMD may utilize one or more post ventricular atrial refractory period (PVARP) blanking intervals to manage blanking for a sensing circuit following a preceding intrinsic ventricular event. As another example, the IMD may manage pacing based on analysis of SCG signals.

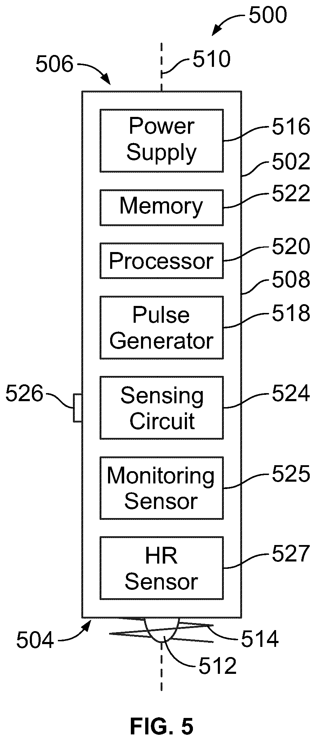

Figure 5 illustrates a side view of the IMD 500 according to an embodiment. The illustrated IMD 500 includes a schematic representation of some internal components of the IMD 500. The housing 502 of the IMD 500 includes a first mounting end 504, an opposite second end 506, and an intermediate shell 508 extending between the first end 504 and the second end 506. The shell 508 is elongated and tubular in shape and extends along a longitudinal axis 510. The mounting end 504 mounts to tissue of an intra-cardiac wall within a chamber of the heart.

The mounting end 504 includes an electrode 512 securely attached thereto and projecting outward from the mounting end 504. The shell 508 includes one or more electrodes 526 provided therein remote from the electrode 512. The electrodes 512 and 526 cooperate to define a sensing vector and to sense local CA signals. The electrodes 512 and 526 are further configured to deliver stimulation energy to tissue of interest. As used herein, "tissue of interest" refers to intra-cardiac tissue that the IMD 500 is configured to monitor and provide stimulation energy. In the illustrated embodiment, the IMD 500 is configured to be affixed directly to the tissue of interest, as described below. The electrode 512 may be a cathode electrode that is actively fixated to the myocardium, while the electrode 526 is an anode electrode. The stimulation energy may be in the form of low-energy pacing pulses, higher-energy shocking pulses, or the like.

When the mounting end 504 is mounted to the intra-cardiac tissue, the electrode 512 is securely affixed to and engages the tissue of interest to deliver the stimulation energy directly thereto. In addition to delivering stimulation energy, in an alternative embodiment the electrode 512 may be used to sense electrical activity from the tissue of interest. The electrode 512 may be formed as a single conductive bulb or, alternatively, as a cone, a single wire, or the like. Optionally, the electrode 512 is not covered with insulation material and the conductive material is exposed to facilitate a good electrical connection to the local wall tissue. Alternatively, at least a portion of the electrode 512 is covered with insulation to prevent electrical conduction to tissue that engages the insulation.

The mounting end 504 includes a fixation element to secure the IMD in or proximate to a local chamber of the heart. For example, the fixation element may be a fixation screw 514 securely attached thereto and projecting outward from the mounting end 504. The fixation screw 514 is configured to extend into the tissue of interest to anchor the IMD 500 to the intra-cardiac tissue. The fixation screw 514 is configured to be screwed into the tissue to firmly adhere the IMD 500 thereto by pressing the mounting end 504 against the tissue and rotating the IMD 500 in a first, coupling direction. The fixation screw 514 may be extracted from the tissue by rotating the IMD 500 in an opposite, uncoupling direction in conjunction with a slight tugging force directed away from the myocardial wall. The fixation screw 514 may be shaped as a helical corkscrew that defines a center channel. For example, the fixation screw 514 may surround the electrode 512 such that the electrode 512 is within the center channel. In an alternative embodiment, the fixation screw 514 is part of the electrode 512. For example, the electrode 512 may have helical threads on an outer surface of the electrode 512, such that the electrode 512 forms the fixation screw 514.

Additionally or alternatively, the fixation element may include one or more loops, tabs and the like that are configured to retain the IMD in a vessel, septal wall, or other tissue proximate to the local chamber of the heart. For example, the IMD and/or fixation element may be formed as described in one or more of U.S. Pat. Pub. No. 2019/0099087, publishing April 4, 2019, and titled WIRELESS SENSOR FOR MEASURING PRESSURE; U.S. Patent 9,993,167, issuing June 12, 2018 and titled APPARATUS AND METHOD FOR SENSOR DEPLOYMENT AND FIXATION; U.S. Published Application 2016/0007924, publishing January 14, 2016, and titled IMPLANTABLE PRESSURE TRANSDUCER SYSTEM OPTIMIZED TO CORRECT ENVIRONMENTAL FACTORS.

The housing 502 retains a power supply 516 and various electronic components that receive electrical current from the power supply 516. The electronic components provide the functionality of the IMD 500, such as controlling the stimulation energy delivered to the electrode 512 and sensing the depolarization along the tissue of interest in response to a pacing pulse or to an intrinsic heartbeat. The power supply 516 stores charge for gradual disbursal to the electronic components as needed. The power supply 516 may be a battery. The power supply 516 has a fixed amount of charge at full capacity. The power supply 516 may be rechargeable in some embodiments and may not be rechargeable in other embodiments. The power supply 516 is fully retained within and surrounded by the housing 502.

The electronic components include a pulse generator 518, a processor 520, a memory 522, a sensing circuit 524 and a monitoring sensor/system 525, such as monitoring system 300 (Figure 3) and/or physiologic sensor 270 (Figure 2). The illustration is intended as an overview of the electronic components only, and the electronic components according to an embodiment of the IMD 500. The pulse generator 518 provides stimulation energy to the electrode 512 which is delivered to the tissue of interest that the electrode 512 engages. The pulse generator 518 includes circuitry to control the output of stimulation energy directed to the electrode 512. For example, the pulse generator 518 produces lower energy pulses for pacing and higher energy pulses for shocking.

As discussed with respect to the IMD 100, the processor 520 of IMD 500 receives SCG signals detected by a heart sound sensor 270, such as the monitoring sensor 525. The SCG signals include heart sound signals for a series of heartbeats over a first time period. The processor 520 segments the SCG signals into SCG segments for corresponding heartbeats within the series of heartbeats over the first time period, and calculates a template based on a first subset of the SCG segments. The processor 520 compares at least a portion of a second subset of the SCG segments to the template to determine matching scores, and can generate an alert in response to a select number of the second subset of SCG segments having the matching scores that satisfy a matching threshold.

The processor 520 is a controller that controls the flow of charge between the power supply 516, the electronic components (such as the pulse generator 518, monitoring sensor 525 and the sensing circuit 524), and the electrodes (such as electrode 512). For example, the processor 520 controls the timing and intensity or magnitude of the stimulation pulses. If multiple electrodes are used to deliver stimulation energy to the intra-cardiac tissue, the processor 520 may synchronize the delivery of the pulses. The processor 520 is communicatively coupled to the pulse generator 518, the sensing circuit 524, the memory 522, and the power supply 516. The processor 520 also functions based on instructions stored locally in the memory 522. The memory 522 is a non-transitory tangible computer readable storage medium. The memory 522 stores programmable and executable instructions for the processor 520. The processor 520 is responsive to the programmable instructions to control operation of the IMD 500 as described herein. The memory 522 may also store data. Some of the data may be stored prior to completing assembly of the IMD 500, while other data may be stored during use of the implanted IMD 500. For example, the memory 522 may be used to store data on intrinsic electrical activity within the heart as monitored by the sensing circuit 524, data on the number, time, and/or magnitude of pacing pulses generated by the pulse generator 518, or the like. The memory 522 is further configured to store HS signals such as SCG signals, EGM signals, COIs, templates, thresholds such as matching thresholds, counters, limits, and the like.

The sensing circuit 524 is configured to monitor intrinsic electrical CA signals within the heart. The sensing circuit 524 is communicatively coupled to one or more sensing electrodes 512, 526 located on or extending from the housing 502. The sensing electrodes 526 are shown located along one or more sides of the shell 508 but may additionally or alternatively be located along the second end 506 of the housing 502. The sensing electrode 526 senses electrical activity, such as physiologic and pathologic behavior and events, and provides sensed signals to the sensing circuit 524 in response. In an alternative embodiment, the pulsing electrode 512 doubles as a sensing electrode, such that the pulsing electrode 512 is used to deliver stimulation pulses and, in-between pulses, monitors the electrical activity within the tissue of interest for the sensing circuit 524.

Optionally, the IMD 500 may include a heart rate (HR) sensor 527 configured to obtain HR data indicative of a patient's heart rate. For example, the HR sensor 527 may sense a blood temperature indicative of a core body temperature of the patient. The processor 520 is further configured to produce a relative temperature signal based on the blood temperature signal. The processor 520 further produces a moving baseline temperature signal based on the relative temperature signal, produces a proportional response signal based on the relative temperature signal and the moving baseline temperature signal, and produces a sensor indicated rate response signal based on the proportional response signal and a base rate. The sensor indicated rate response signal can also be based on a dip response signal and/or a slope response signal. When in a therapy mode, the processor 520 is configured to adjust the at least one of the TR delay or SR BI based on the HR data. For example, the processor 520 may be configured to adjust one or more pacing parameters to control a pacing rate based on the sensor indicated rate response signal. For example, the processor 520 may adjust the AV delay, VV delay, PVARP blanking period and the like, based on the sensor indicated rate response signal.

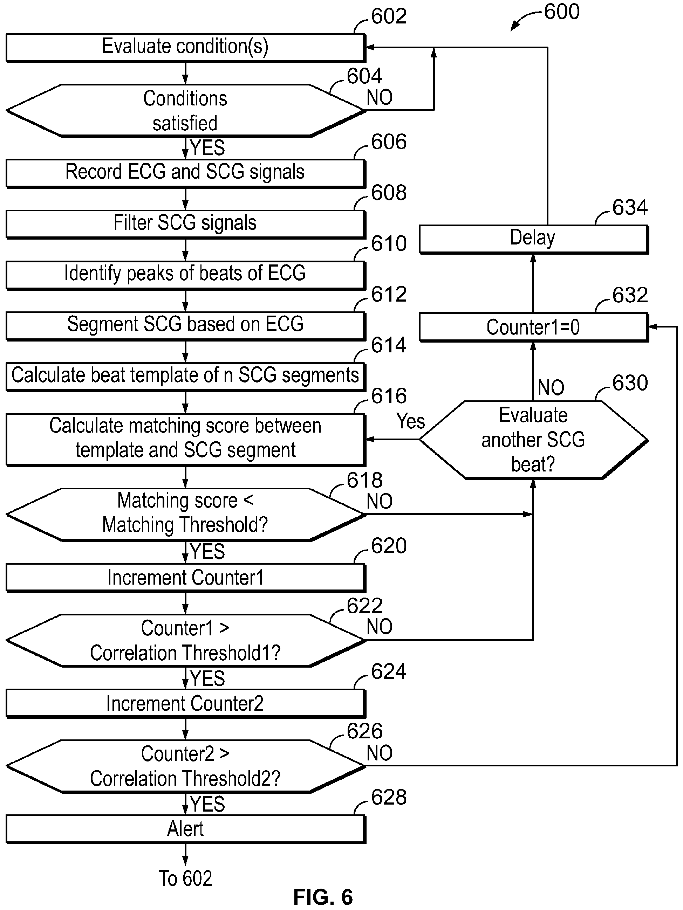

Figure 6 illustrates a computer-implemented method 600 that discriminates between patients with VHD and/or PHV dysfunction and healthy patients in accordance with embodiments herein. For example, the method evaluates relatively short time periods of SCG signals to detect valve dysfunction (e.g., mitral, tricuspid, pulmonary, aortic) and/or dysfunction of a prosthetic heart valve. For each time period, the method calculates a template based on a plurality of SCG segments to which at least a portion of the remaining SCG segments are compared. Patients with VHD or PHV dysfunction will have a higher beat-by-beat variability, resulting in a lower matching score than healthy patients. An alert is generated if a certain number of SCG segments have a matching score that does not satisfy a matching threshold; that is, a predetermined number of SCG segments are a relatively poor match compared to the template.

The operations of Figure 6 may be implemented by hardware, firmware, circuitry and/or one or more processors housed partially and/or entirely within an IMD 100, 500, an external device 154, external monitor 156, wearable device, a local external device, remote server or more generally within a health care system. Optionally, the operations of Figure 6 may be shared across devices, and thus partially implemented by one or more of an IMD 100, 500, an external device 154, external monitor 156, wearable device, a local external device, remote server or more generally within a health care system. For example, the IMD 100, 500 includes IMD memory and one or more IMD processors, the external device 154 includes external device memory and one or more external device processors, and the external monitor 156 includes external memory and one or more external processor, and further, other of the external devices/systems (e.g., local, remote or anywhere within the health care system) that may implement the operations of Figure 6 include external device memory and one or more external device processors.

Although the method of Figure 6 is written primarily from the viewpoint of the IMD 100, 500, it should be understood that after signal data, such as the ECG and SCG signals are acquired, the signals can be transmitted to an external device or a different implanted device for evaluation/processing/storage.

At 602, one or more processors evaluate certain physical conditions of a patient to determine whether conditions are appropriate for evaluating the SCG signal. For example, the one or more processors may only activate the method when the patient is asleep, during periods of inactivity, during periods of predetermined postures, and the like. Sleep, activity, and posture can be determined by the sensor 270, monitoring sensor 525, etc.

Due to the noisy nature of the HS signal (e.g., SCG signal) the patient should be at rest (not moving) during the data collection. To determine when the patient is at rest, the IMD 100, 500 may use a sleep detection feature. The sleep detection feature can use 1D activity and trended posture measurements to determine if the patient is in low activity, recline or lying down state. In one example, the sleep detection feature is a processor that continuously obtains accelerometer data related to the movement, posture, or otherwise related to a patient and analyzes the accelerometer data to determine that a patient has been laying down for a determined period of time.

At 604, the one or more processors determine if the physical condition(s) have been met. If no, the method returns to 602. If the physical condition(s) are met, flow passes to 606.

At 606, the one or more processors simultaneously acquire and/or record the ECG or IEGM (collectively referred to herein as ECG signals) and SCG signals. The acquisition of the ECG and SCG signals can be in response to a trigger when the condition(s) are met. In some embodiments, "N" beats can be recorded, wherein N = 30 beats, 60 beats, 30-60 beats, etc., and span an associated acquisition time period. In other embodiments, the ECG and SCG signals are acquired for an acquisition time period, such as 30 seconds, 60 seconds, 30-60 seconds, based on heart rate, etc., and include the number "N" beats within the time period.

At 608, the one or more processors filter the SCG signals. For example, the SCG signals can be filtered based on frequency, i.e., 7.5 Hz -100 Hz or 15 Hz - 100 Hz and a selection of one of the x/y/z axes. In some embodiments the SCG signals can be filtered based on the breathing cycle, such as to remove any motion artifact caused by patient breathing.

In some embodiments, the ECG and SCG signals may be recorded and stored in a loop recorder. In this case, the one or more processors can access the collection interval of interest or time period for processing.

At 610, the one or more processors identify one or more COI within the ECG signal. In this example, a peak, and specifically the R-wave peak, is used to identify the heartbeat. In some embodiments, the R-wave peak in the ECG signal is identified and is used as a reference for segmenting the SCG signal. In other embodiments, other characteristics of interest may be identified within the ECG signal and used to segment the SCG signal.

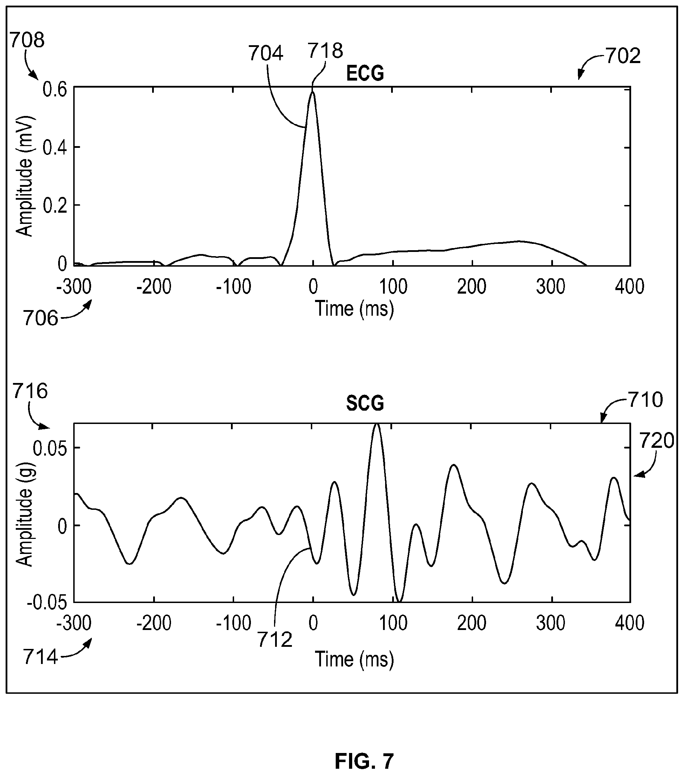

Figure 7 illustrates an example of an electrocardiogram (ECG) signal and a seismocardiogram (SCG) signal of a heartbeat that have been simultaneously acquired in accordance with embodiments herein. In this example, the ECG signal has been recorded via 1-lead and the SCG signal has been acquired via an accelerometer (e.g., sensor 270, 525). The ECG and SCG signals can be acquired using any of the IMDs described herein and/or by external monitor(s) and devices such as a Holter monitor, sensors within a garment/strap, an application via a smartphone, etc. The ECG and SCG signals are representative of a patient with heart valve disease (VHS).

In top panel 702 showing ECG signal 704, horizontal axis 706 plots time in milliseconds (ms) while vertical axis 708 plots amplitude in millivolts (mV). Bottom panel 710 shows SCG signal 712, horizontal axis 714 plots time in milliseconds (ms), and vertical axis 716 plots amplitude in units of gravitational constant (g). The horizontal axis 706, 714 for both the top and bottom panels 702, 710 illustrates the same time or segmentation window. The segmentation window for delimiting heartbeats in this example is the interval from 300 ms before to 400 ms after R-wave peak 718 of the ECG signal 704, as discussed below. It should be understood that the segmentation window can extend more or less than 300 ms before the R-wave peak 718 and more or less than 400 ms after the R-wave peak 718. The bottom panel 710 thus displays an SCG segment 720. For example, the segmentation window can be defined to include at least the SCG signal data corresponding to heart valve and prosthetic heart valve sounds of interest.

At 612 of Figure 6, the one or more processors temporalize and segment the SCG signal 712 into a series of SCG segments 720 over the time period based on the ECG signal 704. In this example, the SCG signal 712 will be segmented into N SCG segments 720, corresponding to N beats. Each beat is defined based on the ECG signal 704 in a time window depending on the R-wave peak 718. The time window is the interval from x ms (e.g., 300 ms) before the R-wave peak 718 to y ms (e.g., 700ms) after the R-wave peak 718. The time window will be normalized with respect to heart rate, and thus may be longer or shorter than shown in Figure 7, and will include at least the heart sound data associated with the valve(s) and/or prosthetic valve(s) of interest.

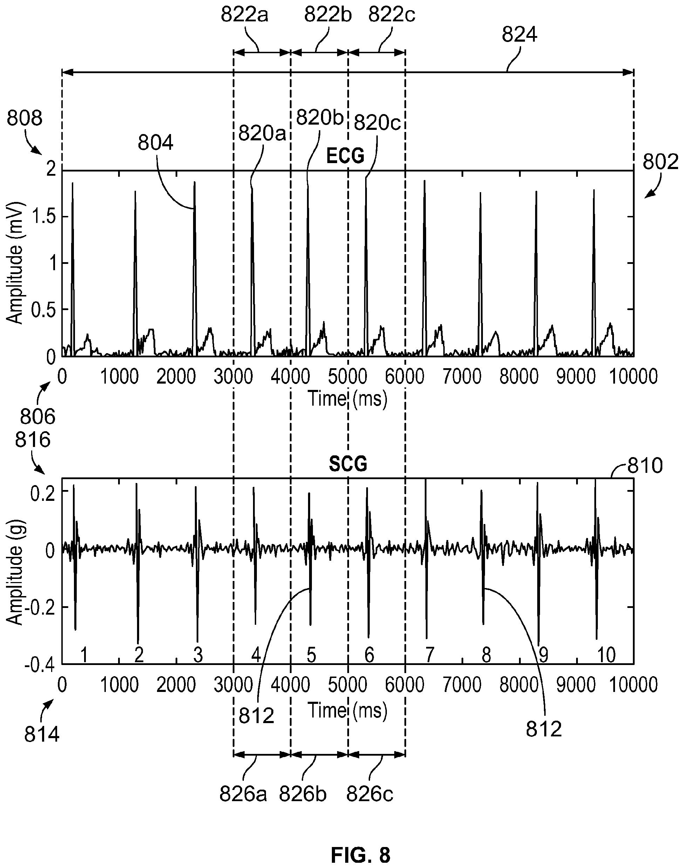

Figure 8 illustrates ECG beats selected as reference for segmenting the SCG signal in accordance with embodiments herein. Again, top panel 802 shows beats of ECG signal 804, wherein horizontal axis 806 plots time (ms) and vertical axis 808 plots amplitude (mV). Bottom panel 810 shows ten consecutive SCG segments of SCG signal 812; horizontal axis 814 plots time (ms) and vertical axis 816 plots amplitude (g).

The one or more processors determine the r-wave peaks 820a, 820b, 820c (not all are individually indicated) for a subset 824 of the acquisition time period. In this example, the subset 824 is 10,000 ms, while the acquisition time period may be 20,000 ms, 30,000 ms, etc. The one or more processors define x ms before the R-wave peaks 820 and y ms after the R-wave peaks 820 as the time window 822a, 822b, 822c (not all are individually indicated) for each beat. In some embodiments, the time window 822 will be applied to define time windows for other beats (not shown) acquired over the acquisition time period. For example, the length of the time window 822, and x ms and y ms, may vary from one ECG acquisition period to another, such as may be recorded two hours later, due to a patient's variability in heart rate. It should be understood that the positions of the vertical lines shown at 1000 ms increments in Figure 8 are for ease of description purposes only and are not limiting, as the time windows 822 are defined by the periods of time before (x ms) and after (y ms) the R-wave peaks.

The one or more processors then define, for the same time windows 822, the SCG segments 826a, 826b, 826c (not all are individually indicated). SCG segments 826 are defined for the entire acquisition time period (not shown).

In some embodiments, the selected ECG beats can be the first n beats of the acquisition time period, although the method is not so limited. In some cases, the selected ECG beats can be later in the acquisition time period, the last beats in the acquisition time period, or non-consecutive beats within the acquisition time period.

At 614, the one or more processors calculate a beat template based on a first subset of the SCG segments 826. For example, the first subset may include the first n SCG segments within the series of N SCG segments, wherein n < N. In some embodiments, n = 10 SCG segments while N equals 30 SCG segments. The n SCG segments (e.g., the first subset) are averaged to form the template. In another example, the first subset can include more or less than ten SCG segments, and N can include more or less than 30 SCG segments.

At 616, the one or more processors calculate a matching score, such as a percentage, between the template and a first SCG segment 826 that is within a second subset of the N SCG segments (e.g., n+1 if the first subset (n) includes the first n SCG segments of N). The matching score can be a percentage based on the entire signal morphology or based on morphological features identified in the SCG signal. In some embodiments, amplitude, peak amplitude, area under the curve, width of signal, difference of peak-to-peak amplitudes between the smallest SCG and the largest SCG, the standard deviation of the width of the SCG signal, and/or intervals between R-wave peak to some SCG peaks can be used to determine a level of correlation between the template and the SCG signal of the compared SCG segment.

At 618, the one or more processors determine whether the matching score satisfies a matching threshold. In some embodiments, the matching threshold can be 70% correlation (e.g., at least 70% correlation, 71%, 72%, 69%). In this example, if the matching score is less than the matching threshold, the threshold is not satisfied, process flows to 620, and the one or more processors increment a first counter, Counter1. Counter1 includes a count of the SCG segments within the current acquisition time period that have poor correlation with the template. If the matching score satisfies the matching threshold at 618, the SCG segment is similar to the template, and flow passes to 630.

At 622, the one or more processors determine whether Counter1 satisfies (e.g., is greater than) a Correlation Threshold1. The Correlation Threshold1 is satisfied when a select number of SCG segments 826 within the current time period satisfies a predetermined threshold. In some embodiments, the Correlation Threshold1 can be set to 10. If Counter1 satisfies or exceeds the Correlation Threshold1, i.e., more than 10 SCG segments within the time period have a matching score that is less than the matching threshold, process passes to 624. If Counter1 does not satisfy the Correlation Threshold1, flow passes to 630.

At 624, the one or more processors increment a second counter, Counter2. Counter2 tracks a number of the SCG segments 826 in excess of Counter1 that have poor correlation with their associated template within an extended time period. The extended time period can be set by a medical practitioner, and can be over a sleeping session of the patient (e.g., eight hours from the first acquisition after conditions are satisfied), 24 hours, 48 hours, one week, one month, etc, In other embodiments, the extended time period can equal the acquisition time period (e.g., 30 second, 60 second). After the extended time period has expired, the one or more processors can set the Counter2 to zero.

At 626, the one or more processors determine whether Counter2 satisfies a Correlation Threshold2 (e.g., more than 12 SCG segments 826 within the extended time period, more than 15 SCG segments within the extended time period, more than 20 SCG segments within the extended time period). If Counter2 does not satisfy (e.g., is less than) the Correlation Threshold2, flow passes to 632. In some cases, less than all of the SCG segments 826 within a time period may be evaluated. If Counter2 satisfies the Correlation Threshold2, flow passes to 628.

In some embodiments, the Counter2 can be incremented a preset (programmable) number of times per time period, such as one, two, three, more than three, etc., before flow passes to 632. If the preset number of times is not satisfied, flow passes to 630.

At 628, the one or more processors generate an alert for suspicion of VHD or PHV dysfunction. For example, a transceiver can be directed to wirelessly transmit an alert to an external device 154. In some cases, some or all of the applicable raw and/or processed SCG signals and CA signals (such as the CA signals the ECG signals are based on) can be transmitted to the external device. The external device can activate an alert, such as a text message, instant message, email, voicemail, etc., update a patient record, etc., to alert the medical practitioner, transmit the data/alert to other computing systems, and the like, such as to a patient care network, such as the Merlin.net.TM. patient care network operated by Abbott Laboratories (headquartered in the Abbott Park Business Center in Lake Bluff, Ill.). In some cases, the one or more processors can transmit the alert and/or signals to a computer system that processes the data using artificial intelligence. After an alert is generated, in some embodiments, the one or more processors set both Counter1 and Counter2 to zero and flow passes to 602.

In another embodiment, at 628, the one or more processors generate an alert in response to a select number of the SCG segments having matching scores that satisfy a matching threshold. In this example, the satisfaction of the matching threshold indicates that the select number of SCG segments have poor correlation with the template. The alert can be processed/transmitted as described above. In still further embodiments, the alerts can be used to generate a heart failure diagnosis.

At 630, the one or more processors determine whether there is another SCG segment 826 (i.e. up to N) to evaluate. If there is another SCG segment to evaluate, flow passes to 616. The template is compared with the remaining SCG segments as discussed, and a matching score (e.g., percentage based on signal morphology or identified morphological features) is determined for each SCG segment.

When the matching score has been evaluated for all SCG segments and an alert has not been raised, the one or more processors reset Counter1=0 at 632.

At 634, the one or more processors monitor for a delay before passing to 602 for the next iteration. For example, the delay can be based on heartbeats, such that M heartbeats (e.g., M = 7200 heartbeats) are acquired before patient condition(s) are evaluated, new signals are recorded, and the algorithm applied again. In other embodiments, the delay can be based on a predetermined time.

In some embodiments, after an extended time period T (e.g., T = 3 days) without an alert activation, the one or more processors can reset Counter2 to 0 or decrease the Correlation Threshold2 in order to maximize the specificity. The one or more processors may track the extended time period T during the time delay 634. In still further embodiments, the one or more processors can generate a message to indicate normal function has been detected for the extended time period T or other defined time period. For example, VHD and/or PHV dysfunction may not exhibit at a level of concern during one or more of the acquisitions, and the extended time period T and/or counters may be adjusted to adjust the specificity.

It should be understood that instead of calculating matching scores between the template and each SCG segment serially, the one or more processors can calculate the matching scores for all of the SCG segments not used to calculate the template substantially simultaneously. The one or more processors can then determine whether the conditions of the Correlation Threshold1 and Correlation Threshold2 are satisfied. Additionally or alternatively, a single correlation threshold and counter may be used.

In some embodiments, thresholds and counters are defined to maximize sensitivity and/or specificity, such as after a clinical evaluation of the patient and/or the results of the algorithm (e.g., too many alerts, too few alerts). For example, a medical practitioner can transmit, via an external device 154 to the IMD 100, 500, an adjustment to one or more of the thresholds, counters, time delays, preset acquisition time windows, and patient conditions, such as after reviewing one or more alerts and/or associated signals.

In accordance with new and unique aspects herein, the method provides the particular treatment and prophylaxis of determining whether the patient is experiencing VHD or PHV dysfunction prior to treating the patient, and thus the patient is spared unnecessary and/or painful treatment, and/or the patient receives improved treatment. Further, as the method accurately detects VHD and/or PHV dysfunction, and thresholds can be adjusted remotely to maximize sensitivity and/or specificity, the patient can be treated more quickly and spared additional exams and time spent in clinic, preventing unnecessary patient stress and anxiety, and saving time and money for the patient, the medical staff, insurance companies and/or government agencies.

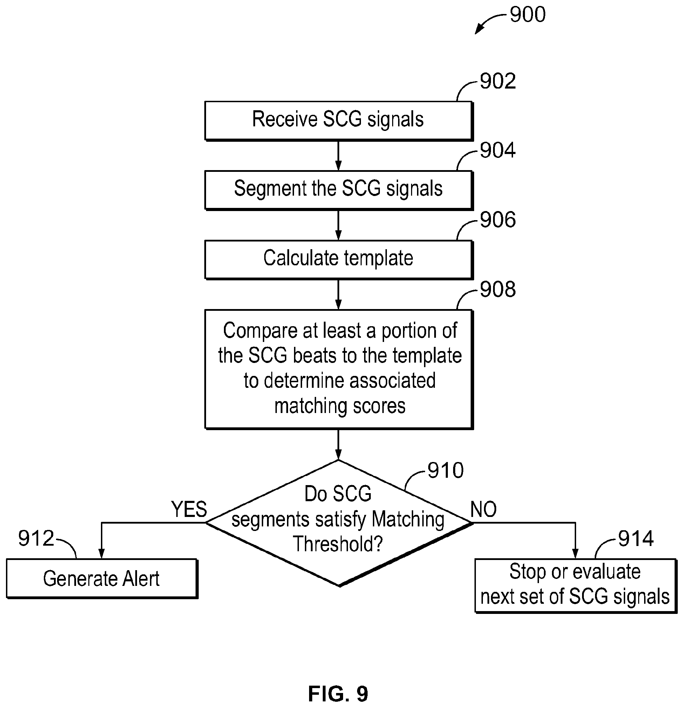

Figure 9 illustrates another method 900 that discriminates between patients with VHD and/or PHV dysfunction and healthy patients in accordance with embodiments herein. Although Figure 9 is primarily discussed from the perspective of an external device, the method is not so limited. For example, the method evaluates relatively short time periods of SCG signals to detect valve dysfunction (e.g., mitral, tricuspid, pulmonary, aortic) and/or dysfunction of a prosthetic heart valve. For each time period, the method calculates a template based on a plurality of SCG segments to which at least a portion of the remaining SCG segments are compared. Patients with VHD or PHV dysfunction will have a higher beat-by-beat variability, resulting in a lower matching score than healthy patients. An alert is generated if a select number of SCG segments have a matching score that indicates that the SCG segments are a relatively poor match compared to the template.

The operations of Figure 9 may be implemented by hardware, firmware, circuitry and/or one or more process ors housed partially and/or entirely within an IMD 100, 500, an external device 154, external monitor 156, wearable device, a local external device, remote server or more generally within a health care system. Optionally, the operations of Figure 9 may be shared across devices, and thus partially implemented by one or more of an IMD 100, 500, an external device 154, external monitor 156, wearable device, a local external device, remote server or more generally within a health care system. For example, the IMD 100, 500 includes IMD memory and one or more IMD processors, the external device 154 includes external device memory and one or more external device processors, and the external monitor 156 includes external memory and one or more external processor, and further, other of the external devices/systems (e.g., local, remote or anywhere within the health care system) that may implement the operations of Figure 9 include external device memory and one or more external device processors.

At 902, the one or more processors receive SCG signals. For example, a certain number of beats (N beats) of SCG signals can be received and/or a certain time period of SCG signals can be received. In some cases, the one or more processors can identify, within an extended recording of SCG signals, a desired time period and/or number of beats to be processed. In some embodiments, corresponding CA signals, such as ECG or EGM signals, can also be received. In further embodiments, information concerning certain physical conditions of the patient when the SCG and ECG/EGM signals were acquired can also be received, and the one or more processors can determine whether conditions are appropriate for evaluating the SCG signal (e.g., sleep, activity, posture).

At 904, the one or more processors segment the SCG signals into a series of SCG segments over the time period. The segmentation can be accomplished based on the CA signals, such as based on one or more features or COI such as R-wave peaks. In other embodiments, morphological features of the SCG signals, such as S1, S2, etc., can be used to accomplish the segmentation.

At 906, the one or more processors calculate a beat template based on a first subset of the SCG segments. For example, the first subset may include n beats within the series of N SCG segments, wherein n < N. In some embodiments, n = 10 SCG segments while N equals 30 SCG segments. The n SCG segments (e.g., the first subset) are averaged to form the template. In another example, the first subset can include more or less than ten SCG segments, and N can include more or less than 30 beats.

At 908, the one or more processors compare at least a portion of a second subset of the SCG segments to the template to determine matching scores. The first and second subsets can include different beats within the time period. The one or more processors calculate a matching score, such as a percentage, between the template and an SCG segment. The matching score can be a percentage based on the entire signal morphology or morphological features identified in the SCG signal. In some embodiments, amplitude, peak amplitude, area under the curve, width of signal, difference of peak-to-peak amplitudes between the smallest SCG and the largest SCG, the standard deviation of the width of the SCG signal, and/or intervals between R-wave peak to some SCG peaks can be used to determine a level of correlation between the template and the SCG segment.

At 910, the one or more processors determine whether a select number of SCG segments have matching scores that satisfy a matching threshold. In this example, the satisfaction of the matching threshold indicates that the select number of SCG segments have poor correlation with the template.

If yes, at 912 the one or more processors generate or declare an alert. The alert may be displayed on a display of the external device, transmitted via text message, instant message, email, voicemail, etc. The one or more processors can update a patient record, etc., to alert the medical practitioner, transmit the data/alert to other computing systems, such as to a patient care network, and/or process the data using artificial intelligence.

If the matching threshold is not satisfied at 910, at 914 the one or more processors stop the process or evaluate the next set of SCG segments.

In some embodiments, the process may utilize more than one threshold as discussed in Figure 6. For example, a counter can maintain a count of SCG segments that have matching scores that satisfy or do not satisfy the matching threshold over multiple time periods.

In some embodiments, the external device may receive SCG signals and, if applicable, corresponding ECG signals, that have been acquired over an extended time period. For example, the SCG signals and ECG signals may be acquired by an IMD 100, 500 implanted within a patient, and the patient may utilize their external device 154 to upload acquired data daily or weekly. When received, the data may be processed serially as discussed in Figures 6 and/or 9, or some or all of the time periods may be processed substantially simultaneously.

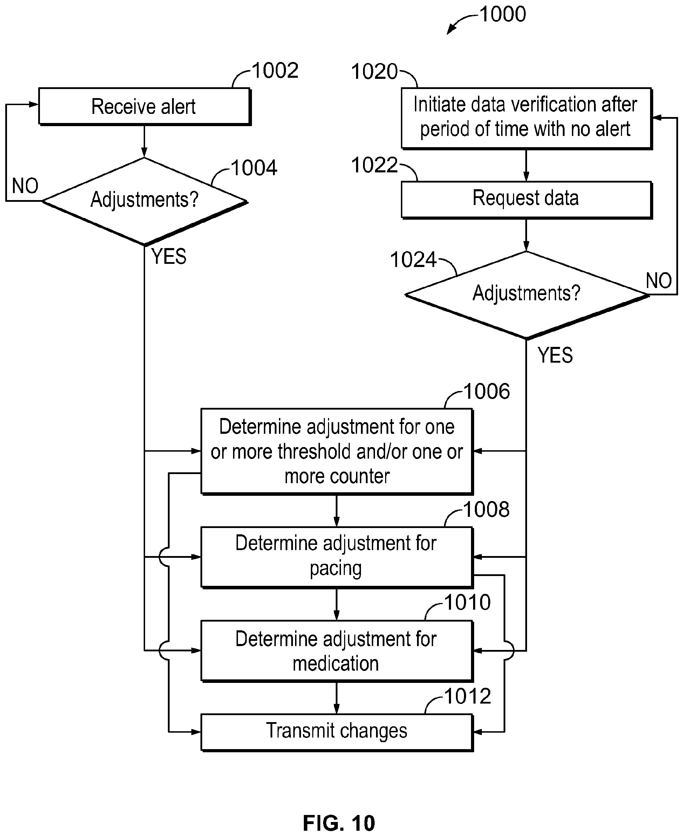

Figure 10 illustrates examples of methods 1000 for treating a patient being monitored for VHD and/or PHV dysfunction in accordance with embodiments herein. Based on the receipt of alerts or the lack of alerts, for example, the method determines whether adjustments can/should be made to change the acquisition and/or processing parameters for data, adjust/start/stop treatment of the patient, and the like.

The operations of Figure 10 may be implemented by hardware, firmware, circuitry and/or one or more processors housed partially and/or entirely within an IMD 100, 500, an external device 154, external monitor 156, wearable device, a local external device, remote server or more generally within a health care system. Optionally, the operations of Figure 10 may be shared across devices, and thus partially implemented by one or more of an IMD 100, 500, an external device 154, external monitor 156, wearable device, a local external device, remote server or more generally within a health care system. For example, the IMD 100, 500 includes IMD memory and one or more IMD processors, the external device 154 includes external device memory and one or more external device processors, and the external monitor 156 includes external memory and one or more external processor, and further, other of the external devices/systems (e.g., local, remote or anywhere within the health care system) that may implement the operations of Figure 10 include external device memory and one or more external device processors.

At 1002, one or more processors receive the alert. The alert can be generated by the IMD 100, 500 or an external cardiac monitor 156 as discussed previously. The alert can be monitored by the IMD 100, 500, the external cardiac monitor 156, the external device 154, another remote device, such as an application associated with a physician and/or physician office, and the like.

At 1004, one or more processors determine whether adjustments to the evaluation algorithm or patient treatments are to be made. In some embodiments, the thresholds and/or counters can be compared to predetermined ranges allowable for adjustment. In other embodiments, AI can review the alert and/or history of alerts, the settings of the acquisition device, signals acquired by the acquisition device, data acquired across a cohort of similar patients, etc., and determine, based on machine learning models, convolutional neural networks, and the like whether the alert(s) are valid, and/or whether adjustments to settings and/or treatments should be made. In still further embodiments, medical personnel can review the alert and patient data to determine whether adjustments should be made. If no adjustments are to be made, flow returns to 1002.

If adjustments are to be made, flows passes to one or more of 1006, 1008, and 1010. At 1006, the one or more processors determine adjustment for one or more threshold, one or more counter, one or more time period, one or more delay, etc. As discussed previously, the thresholds and counters may be adjusted within a predetermined range. In some embodiments, the medical practitioner may choose settings that are outside previously set ranges, and/or adjust ranges for future automatic adjustments. In other embodiments, AI can utilize knowledge based on many other patients with similar symptoms and/or pathology and the instant patient history to determine the appropriate settings. These adjustments provide the benefit of either increasing or decreasing the number of alerts, as needed, for patient treatment. In some embodiments, AI can generate alerts/messages to propose settings and/or ranges that are presented to medical personnel via display, email, text, notes made in patient file, etc., decreasing the amount of time medical personnel spend reviewing data, making decisions, etc., and can improve quality by providing a consistent level of care for many patients.

At 1008, the one or more processors determine an adjustment for pacing or other active treatment. In some embodiments, pacing can be adjusted to improve the matching scores of the SCG segments, or correlation of the SCG segments with the template. In other embodiments, a pacing configuration or "sweep" can be used to acquire SCG signals with different pacing settings. The matching scores of the SCG segments acquired with the different pacing settings can be compared to identify the pacing settings that improve the matching scores. For example, the pacing settings can be adjusted within a predetermined range, adjusted within a range identified by AI, etc. In some cases the proposed pacing settings can be sent in an alert to medical personnel for approval.

Alternatively or additionally, the one or more processors determine an adjustment for sensing, such as of the CA signals. In some embodiments, sensing can be adjusted to improve, for example, the detection of one or more COI of the CA signals such as R-wave, P-wave, T-wave, etc. For example, adjusting the sensing to improve the detection of R-waves can improve the identification of the heartbeat, improve the detection of intervals between the R-wave peak to one or more SCG peak, improve the segmentation of the SCG signals based on the CA signals (e.g., ECG), and the like.

At 1010, the one or more processors determine an adjustment for titration of medication. For example, one or more processors evaluate the medication schedule/dosage and adjust delivery of the medication, such as via the medication delivery device 160 (Figure 1). In another example, AI can utilize knowledge based on many other patients with similar pathology and medication(s) and the instant patient history to determine appropriate settings. These adjustments provide the benefit of improved patient outcome by maintaining an appropriate and effective level of medication, and can be accomplished on a more immediate basis and lower cost compared to requiring the patient to schedule a clinic visit for evaluation.

The method flows from 1006, 1008, and/or 1010 to 1012, and the one or more processors transmit changes to the appropriate device(s). For example, pacing changes are transmitted to the IMD 500. Changes to thresholds and counters are transmitted to the IMD 100, 500 and/or external monitors 156, 158, while changes to medication can be sent to an external or implantable medication delivery device 160 that automatically delivers medication. In other embodiments, changes to medication can be communicated to the patient, such as via a patient application, such as if the medication is administered by the patient. It should be understood that some changes to settings and medication may be accomplished via an external device 154 that is capable of programming the applicable device 100, 500, 156, 158, 160.

In accordance with new and unique aspects, a particular treatment for the medical condition of VHD and/or PHV dysfunction is delivered. The treatment is selected based on matching scores of the SCG segments that result in an alert, and the treatment can be delivered via an IMD 100, 500 (e.g., pacing) and/or a medication delivery device 160. The treatment can be delivered, for example, to maximize sensitivity and/or specificity.

In another embodiment, at 1020 the one or more processors can, such as after a predetermined period of time during which no alert has been received, initiate data verification. For example, a shorter predetermined period of time may be set initially while settings are being fine-tuned, while a longer predetermined period of time may be set to confirm that valve issues are being identified.

Optionally, at 1022, the one or more processors can request signal data, data such as historical threshold and/or counter settings, history of pacing and/or medication settings, and/or history of alerts for analysis. In some cases, this information may be available in the patient record.

At 1024, the one or more processors determine whether adjustments should be made, flowing as needed to 1006, 1008, and/or 1010. For example, one or more thresholds and/or counters may be adjusted to a previous setting to increase the number of alerts. Ranges may be adjusted to allow for more or less future automated adjustments. Again, AI can utilize knowledge based on many other patients with similar symptoms and/or pathology, along with instant patient history/condition, to determine the appropriate settings. These adjustments provide the benefit of either increasing or maintaining the number of alerts, as needed for patient treatment.

If no adjustments are to be made at 1024, flow returns to 1020.

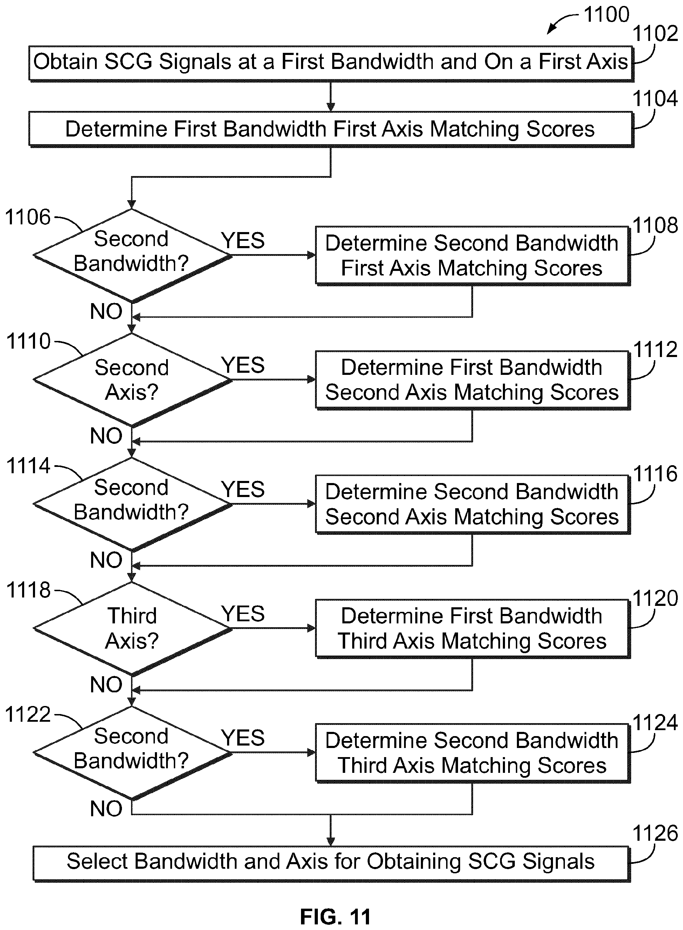

Figure 11 illustrates a method 1100 for obtaining SCG data over different bandwidths and axes and determining which SCG signal to use when diagnosing VHD and/or PHV dysfunction in accordance with embodiments herein. For example, the method may be used when the IMD is initially implanted and/or the IMD may have migrated from the initial implanted position. In one example, migration can be determined using the migration algorithms, systems, and related devices illustrated and described in U.S. Patent Publication No. 2023/0346258, filed March 30, 2023, entitled SYSTEM FOR DETERMINING CHANGE IN POSITION OF AN IMPLANTED POCKET. In another example embodiment, an artificial intelligence algorithm is utilized to determine migration.

The method 1100 is performed so that one or more processors of an IMD 100, 500 can determine the components of a HS sensor (e.g., sensor 270, 525) to utilize to obtain HS data. In one example, the IMD 100, 500 can obtain HS data over two bandwidths and includes a HS sensor 270, 525 that is a three-axis accelerometer that obtains HS signals along a first axis, second axis and a third axis. In an example, a matching score determination can be made related to each bandwidth and each axis of the three-axis accelerometer (e.g., the bandwidth HS components). Therefore, six different matching score determinations can be made. In other examples, the IMD 100, 500 may be able to obtain only one bandwidth, more than two bandwidths, only one axis, two axes, etc. Consequently, the number of matching score determinations that can be made by the one or more processors can depend on the type of IMD and HS sensor used to obtain the HS signals. Still, once each possible matching score determination is made, one can be selected as the selected matching score such that additional HS signals from that point forward are obtained based on the selected matching score. So, if a bandwidth HS component represents a first bandwidth third axis, and that bandwidth HS component results in the best matching score results, the bandwidth HS component representing the first bandwidth third axis is utilized to obtain the additional HS signals.

At 1102, a HS sensor senses and obtains SCG signals within a first bandwidth and along a first axis over a time period or for N beats. In one example, the HS sensor is a three-axis accelerometer, and the first axis is an X-axis. In another example, the first bandwidth is a wideband bandwidth that can have a frequency range of between 7.5-100Hz. In some embodiments, the IMD simultaneously acquires ECG signals that are used to temporalize and segment the SCG signals, while in other embodiments the SCG signals can be segmented based on detected COIs within the SCG signals.

At 1104, one or more processors determine first matching scores based on the SCG segments and template (see Figures 6-10).

At 1106, the one or more processors determine whether the SCG signals can be obtained along the first axis within a second bandwidth. In one example the second bandwidth is a narrowband bandwidth with a frequency range of between 15-100Hz. If the SCG signals can be obtained along the first axis within a second bandwidth, at 1108 the one or more processors obtain the SCG signals on the first axis within the second bandwidth and determine second matching scores.

If at 1106, the one or more processors determine the SCG signals cannot be obtained within the second bandwidth, or after the SCG signals along the first axis within the second bandwidth are obtained, at 1110, the one or more processors determine whether SCG signals can be obtained within the first bandwidth along a second axis. If the SCG signals can be obtained within the first bandwidth along the second axis, at 1112, the one or more processors obtain SCG signals with the first bandwidth along the second axis and determine third matching scores.

After the third matching scores are determined, at 1114, the one or more processors determine whether the SCG signals can be obtained within the second bandwidth along the second axis. At 1116 the SCG signals within the second bandwidth along the second axis are obtained, and the one or more processors determine fourth matching scores.

After the fourth matching scores are determined, at 1118 the one or more processors determine whether the SCG signals can be obtained within the first bandwidth on a third axis. If the SCG signals can be obtained within the first bandwidth on the third axis, at 1120, the one or more processors obtain the SCG signals and determine fifth matching scores.

After the fifth matching scores are determined, at 1122 the one or more processors determine whether the SCG signals can be obtained within a second bandwidth along the third axis. If the SCG signals can be obtained within the second bandwidth along the third axis, at 1124, the one or more processors obtain the SCG signals and determine sixth matching scores.

After SCG signals are obtained at all frequencies, and on all axes, at 1126 the one or more processors analyze the matching scores, such as by comparing each matching score to the matching threshold. For example, the one or more processors can determine the frequency and axis that generates the SCG segments that best satisfy the matching threshold. In some embodiments, the selected frequency and axis can be based on the configuration associated with the lowest Counter2 (see Figure 6), lowest Counter1, configuration that did not generate an alert, etc. The selected frequency and axis may be used for a predetermined time period to collect SCG data, such as a 24-hour period, a week, a month, etc., and/or select and process the SCG data. At the end of the predetermined time period, or when a migration algorithm detects device migration, the one or more processors may repeat the process of evaluating the bandwidth and axes. The SCG signals obtained using the selected frequency and axis are more accurate and can be utilized for diagnosis of patient conditions, determining false determination, or the like.

With respect to the methods and systems utilized to identify and treat VHD and PHV dysfunction disclosed herein, a feasibility evaluation has been conducted utilizing two public databases: a database of measurements collected from 100 pathological subjects affected by one or more VHDs (C. Yang et al., "An Open-Access Database for the Evaluation of Cardio-Mechanical Signals From Patients with Valvular Heart Diseases," Front. Physiol., vol. 12, no. 750221, 2021. https://doi.org/10.3389/fphys.2021.750221), and a further database of measurements collected from 20 healthy subjects (Combined measurement of ECG, Breathing and Seismocardiograms (CEBS) database. https://doi.org/10.13026/C2KW23). Both databases contain SCG and ECG signals acquired simultaneously from subjects at rest, in the supine position. In the VHD patient database (Yang), the signals were acquired at 256 or 512 Hz, while in the CEBS database (García-González) the signals were acquired at 5 kHz. Before processing, all signals were resampled at 1 kHz. Then, for each patient, a morphological correlation index between a template (e.g., average of first 10 SCG segments) and all other SCG segments were calculated to assess the morphological similarity between the template and other SCG segments. Then, the correlation indexes were summarized by calculating the mean and standard deviation (Std).

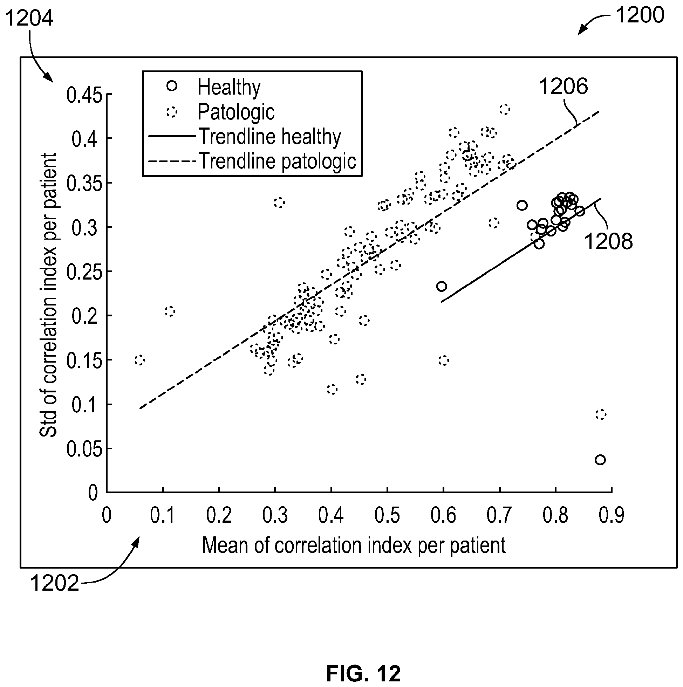

Figure 12 shows a graph 1200 of the mean and standard deviation (Std) of morphological correlation indexes between a template (e.g., average of first 10 SCG segments) and all other SCG segments for a group of 100 pathological and 20 healthy patients. highlighting a substantial difference between them in accordance with embodiments herein. Horizontal axis 1202 plots mean of correlation index per patient while vertical axis 1204 plots Std of correlation index per patient. The trendline equations shown in Figure 12 are:

$$y = 0.40x + 0.07 \text{ (pathological group) (trendline 1206; and}$$

$$y = 0.40x - 0.02 \text{ (healthy group) (trendline 1208).}$$

.

In healthy patients, the mean of correlation indexes was higher, suggesting that the template was well correlated with all other SCG segments (i.e., the signal had high beat-by-beat morphological similarity). In patients with VHD, correlation indexes were either lower or had higher Std than healthy patients, suggesting a worse correlation between the template and all other SCG segments (i.e., the signal had low beat-by-beat morphological similarity). Therefore, accurate recognition of healthy and pathological patients is feasible with proper threshold and counter definitions.

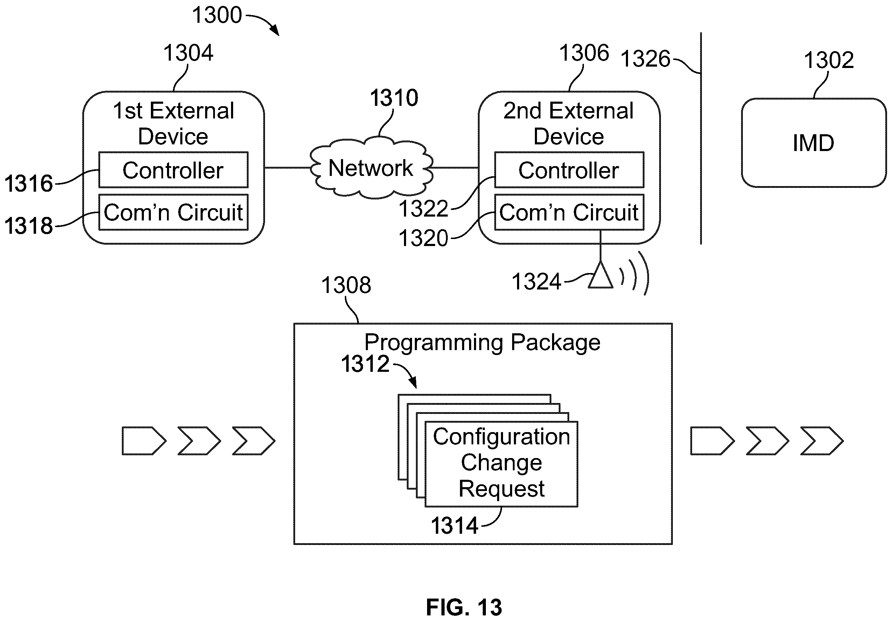

Figure 13 is a schematic illustration of a system 1300 according to an embodiment. In one example, the system 1300 is at least one of the system as illustrated and described in U.S. Pub. No. 2023/0414951, filed June 19, 2023, entitled SYSTEM AND METHOD FOR IMPLANTABLE MEDICAL DEVICE REMOTE PROGRAMMING. The system 1300 provides secure remote programming of an IMD 1302 while the IMD 1302 is implanted within a patient. In addition to the IMD 1302, the system 1300 includes one or more external devices. In the illustrated embodiment, the external devices include a first external device 1304 and a second external device 1306, but the system 1300 may include only a single external device or more than two external devices in other embodiments. The system 1300 may systems, devices, sensors, etc., previously described herein. For example, the IMD 1302 may represent the IMD 100, 500 and the first external device 1304 may represent the external device 154.

The one or more external devices build a programming package 1308 that is designed to update an operating configuration of a programming session of the IMD 1302. The operating configuration of the programming session of the IMD 1302 generally refers to the specific settings, functions, parameters, and the like that dictate the device behavior. For example, the operating configuration affects how the IMD 1302 collects data, such as cardiac signals, how the IMD 1302 analyzes the data, and how the IMD 1302 responds to the data. The response may include activating the pulse generator or shocking circuit to deliver stimulation therapy to the patient, saving data in the memory device, communicating data to the external device 1306, or the like. Even if the IMD 1302 has been previously programmed, the operating configuration of the programming session may be updated over time due to physiological changes in the patient, unplanned movement of the IMD 1302 within the patient, or even to enhance functionality of the IMD 1302 based on software developments.

In the illustrated embodiment, the first external device 1304 builds the programming package 1308 related to the programming session. In a non-limiting example, the first external device 1304 is accessed and utilized by a clinician to select a collection 1312 of multiple configuration change requests 1314 for inclusion in the programming package 1308. The first external device 1304 may be a clinician programming device, a workstation, or a computing device (e.g., smartphone, tablet computer, laptop computer). The clinician may select the collection 1312 by communicating via a network 1310 with a server or other computing device in the cloud. For example, the clinician may be required to log in to a secure portal prior to receiving access to select the configuration change requests 1314 and command the generation of the programming package 1308. The first external device 1304 may be remote from the second external device 1306 and the patient.

The first external device 1304 conveys the programming package 1308, as a message, to the second external device 1306 via the network 1310. In a non-limiting example, the second external device 1306 is a device that is disposed proximate to the patient, at least at a time that the second external device 1306 communicates the programming package 1308 to the IMD 1302. For example, the second external device 1304 may be a bedside monitoring device or a computing device utilized or possessed by the patient, such as a smartphone, tablet computer, laptop computer, or the like.

When the second external device 1306 receives the programming package 1308, the programming package 1308 may schedule a designated time in the future at which to transmit the programming package 1308 to the IMD 1302. The second external device 1306 initially stores the programming package 1308 in a memory device of the device 1306 until the designated time. At the designated time, the second external device 1306 wirelessly transmits the programming package 1308 to the IMD 1302 to update a programming session. This process of scheduling in advance the delivery of the programming package to the IMD via an intermediary device is referred to as asynchronous remote programming. The IMD 1302 is implanted within the patient at the time that the IMD 1302 receives the programming package 1308. According to embodiments herein, the IMD 1302 utilizes a virtual device engine (VDE) to simulate operation of the IMD based on the programming package. The VDE communicates with the IMD to ensure the most recent programming configuration is known. In an example, the VDE can verify whether the programming package is configured and based on the most recent and most updated programming session at the IMD. If all the elements of the programming package 1308 are verified, the IMD 1302 executes all the configuration change requests 1314 to update the operating configuration of the IMD 1302. On the other hand, if at least one of the elements are not verified, as described herein, the IMD 1302 does not execute any of the configuration change requests 1314, such that the operating configuration of the IMD 1302 is not updated based on the change request.

The first and second external devices 1304, 1306 include respective controller circuits (e.g., controllers) and communication circuits. Each of the controllers includes one or more processors. In the process described above, the controller 1316 of the first external device 1304 generates the programming package 1308. The communication circuit 1318 of the first external device 1304 communicates the programming package 1308 to the second external device 1306 via the network 1310. The communication circuit 1320 of the second external device 1306 receives the programming package 1308 and conveys the package 1308 to the controller 1322 of the second external device 1306 for analysis. The controller 1322 determines the scheduled delivery time and transmission characteristics, such as frequency channel and protocol, for communicating the programming package 1308 to the IMD 1302. At the scheduled delivery time, the controller 1322 utilizes the communication circuit 1320 to transmit the programming package 1308 to the IMD 1302. The communication circuit 1320 may include an antenna 1324 that wirelessly transmits the programming package 1308 via RF signals, such as Bluetooth. The signals that represent the programming package 1308 penetrate the body 1326 of the patient and reach a receiver of the IMD 1302. The receiver may be a communication modem, which conveys the received data packets to the one or more processors of the microcontroller for analysis. In this way, the IMD 1302 is beneficially able to receive the programming package 1308 without requiring a network connection or a proximity sensor.

In instances as described above in which the communication of the programming package 1308 from the source to the IMD 1302 is delayed and/or indirect, there is a risk that the contents of the programming package 1308 may be tampered with or corrupted after generation and before receipt by the IMD 1302. The system 1300 and method described herein perform cryptographic operations to ensure that the configuration change requests 1314 are immutable and that the source of the package 1308 is authenticated prior to execution by the IMD 1302.

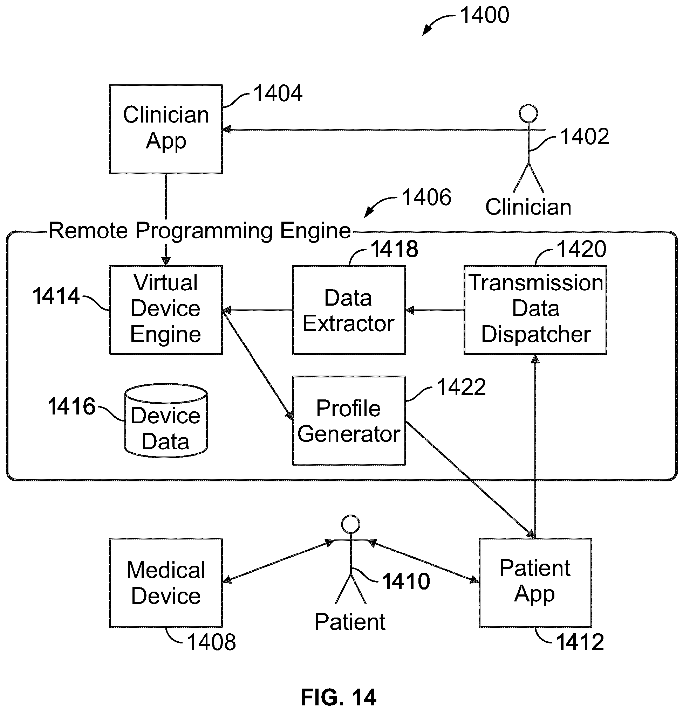

Figure 14 illustrates a system 1400 for verifying an update sent by a clinician 1402 from a clinician application 1404 through a remote programming engine (RPE) 1406 to an IMD 1408 of a patient 1410 that utilizes a patient application 1412. The patient application 1412 in one example can be accessed and programmed on a patient programming device. The update may be an update of a treatment, software, firmware, application, program, etc. The clinician application 1404 may be stored in a storage device of a clinician programming device, including a clinician laptop computer, desktop computer, CPU, smartphone, smartwatch, tablet, or the like. The clinician application 1404 may also be stored within a network, such as in the cloud, at a server, at a network resource, or the like. In each instance, the clinician application 1404 includes instructions that may be executed by one or more processors. The one or more processors can be of the clinician laptop computer, desktop computer, CPU, smartphone, smartwatch, tablet, etc., of a server, of a network device, or the like. Based on the instructions, the steps of a method or process for updating the IMD 1408 are provided.

The RPE 1406 may be in the cloud, in a network, at a server, over Bluetooth (BLE), or the like. The RPE 1406 includes a virtual device engine (VDE) 1414 that can simulate the operation of the IMD 1408. By simulating the operation of the IMD 1408, the VDE 1414 obtains information related to the IMD 1408, including all updates, changes, modifications, etc., that occur at the IMD 1408 over time. The VDE 1414 can store this IMD 1408 operational information at a device data store 1416. In one example, the RPE 1406 includes a data extractor 1418 that extracts such information from a transmission data dispatcher 1420 that continuously receives transmission data from the IMD 1408 via the patient application 1412.

By continuously obtaining the IMD data, the VDE 1414 can continuously simulate the operation and device activities of the IMD 1408, and store data at the device data store 1416. The VDE 1414 can thus manage a communication session, including a programming session, implemented by the clinician 1402 from initialization, to pending, to completed. In one example, when the clinician app communicates the desired modifications, changes, updates, etc., to the RPE 1406, the RPE can synchronize the current state of the IMD 1408 with the modification, change, update, etc., desired to be implemented. The VDE 1414 can provide the synchronized data and information to an asynchronous profile generator 1422 to generate an asynchronous remote programming profile that includes updated program instructions for updating the IMD 1408. In another example, if the IMD 1408 has been modified previously without the knowledge of the clinician 1402, the VDE 1414 ensures the desired update occurs on the most recent version of a program, software, firmware, application, or the like.

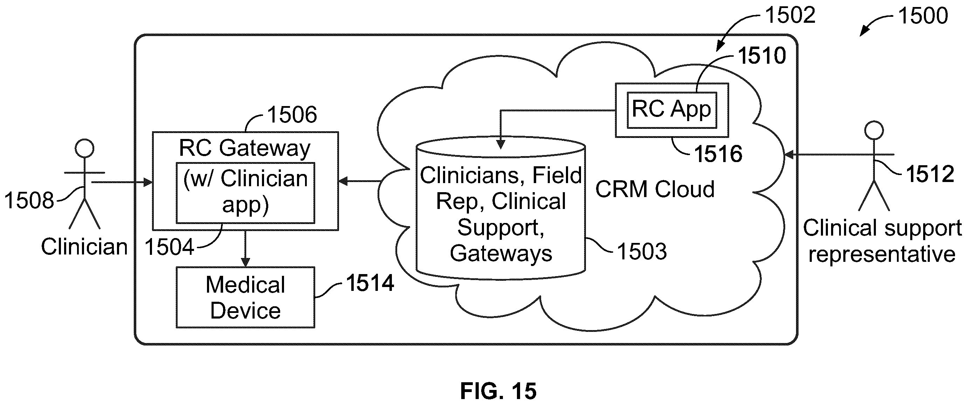

Figure 15 illustrates a medical device remote controlled (MDRC) system 1500 that includes an engine 1502, patient database 1503, a clinician application 1504 that can be accessed at a clinician electronic device 1506 (e.g., gateway) for local users 1508 (e.g., clinicians), an RC application 1510 at a remote electronic device 1516 (e.g., gateway) for RC users 1512 (e.g., clinical support representatives), and at least one medical device 1514. By providing the MDRC system 1500, communication sessions can be provided.

The engine 1502 can include one or more processors for executing instructions to perform processes and methods described herein. The engine 1502 can also include a memory or storage device for storing information, including patient information, medical device information, treatment information, etc. In an example, the engine 1502 is within a cloud environment that can be accessed by numerous electronic devices other than the clinician electronic device 1506 and the remote electronic device 1516. In one example, the engine 1502 is coupled to the patient database 1503 that includes patient information, clinician information, field representative information, clinical support information, medical device information, electronic device information, including electronic device information related to the clinician electronic device 1506 and the remote electronic device 1516, or the like. The information stored can be related to an individual patient, numerous patients, patient medical devices, generic medical devices, studies, tests, other patient information, etc. In all, the patient database 1503 and engine 1502 can be coupled within a network, including a mesh network, cloud network, Bluetooth network, etc. and communicate with plural clinician electronic devices, plural remote electronic devices, etc., to obtain information related to plural patients, medical devices, treatments, or the like. The patient database can include algorithms, including artificial intelligence algorithms for analyzing information obtained from the plural patients, medical devices, treatments, etc., to provide recommendations, modifications, updates, changes, or the like to medical devices, treatments, etc., accordingly.

The clinician electronic device 1506 is the electronic device that is at the location of the patient, and/or medical device 1514. The clinician electronic device 1506 is considered local because the patient, and/or medical device 1514 is at the location of the clinician electronic device 1506 and is not remote from the patient and/or medical device 1514. In one example the medical device 1514 is an IMD. In another example, the medical device may be a neurostimulation device, monitor, or the like. The clinician electronic device can be a laptop computer, desktop computer, mobile device, server, iPad, or the like.

The clinician electronic device 1506 can include one or more processors for executing instructions to perform processes and methods described herein. The clinician electronic device can also include a memory or storage device for storing information, including patient information, medical device information, treatment information, etc. The clinician electronic device can also include an interface so that a local user 1508, such as a clinician, can input information into the clinician electronic device 1506. The interface in one example is an input device that can include a touch screen, keyboard, mouse, microphone, or the like to provide information, commands, etc., to the clinician electronic device 1506. In addition, the clinician electronic device 1506 can include an output device, such as a screen, display, speakers, etc., for providing information and data to the local user 1508. The clinician electronic device 1506 can thus be utilized by the local user 1508 to obtain information directly from the medical device 1514, and/or a patient that has the medical device 1514.

When a local user turns on the clinician electronic device 1506, the clinician electronic device 1506 detects network connectivity and automatically performs an analysis to ensure the engine 1502 is operational. In addition, the analysis can determine the identification, name, serial number, etc., associated with the clinician electronic device 1506, the version of the software, firmware, or the like of the clinician application 1504, operating system, etc., of the clinician electronic device 1506, the location or region of the clinician electronic device 1506, or the like.

In addition, the remote electronic device 1516 is located remotely, or away from the patient and/or medical device 1514. The remote electronic device 1516 can be in a hospital, clinician's office, clinician's home, or the like that is not in the location of the patient and/or medical device 1514. The remote electronic device 1516 can be a laptop computer, desktop computer, mobile device, server, iPad, or the like that includes an interface so that a RC user 1508, such as a clinician support representative, can input information into the remote electronic device 1516. The remote electronic device 1516 in one example is an electronic device of a network.

The remote electronic device 1516 can include one or more processors and a memory for executing instructions and storing information like the clinician electronic device 1506 and engine 1502. In addition, the remote electronic device 1516, like the clinician electronic device 1506, can include an interface that is an input device, along with an output device. The remote electronic device 1516 also includes the RC application 1510 that includes program instructions for obtaining patient information from the patient database 1503 via the engine 1502. The RC application 1510 can also communicate with the clinician application 1504 to obtain physiological information related to a patient that is obtained from the medical device 1514 via the engine 1502.

When a RC user 1508 turns on the remote electronic device 1516, the remote electronic device 1516 detects network connectivity and automatically performs an analysis to ensure the engine 1502 is operational. In addition, the analysis can determine the identification, name, serial number, etc., associated with the remote electronic device 1516, the version of the software, firmware, or the like of the RC application 1510, operating system, etc., of the remote electronic device 1516, the location or region of the remote electronic device 1516, or the like.

Heart valves can become damaged as a natural consequence of ageing, due to rheumatic heart disease, a bacterial infection affecting the valves, and/or as a consequence of a congenital abnormality. In some parts of the world (e.g., Africa), VHD is one of the most common causes of heart failure, whereas in Europe and America, VHD is a rather rare cause of heart failure. However, any abnormality that affects the functioning of the valves may lead to the development of heart failure. In patients with heart failure and a weakened heart muscle (e.g., reduced ejection fraction), enlargement and dilatation of heart cavities can lead to distortion and abnormality of the heart valves. This abnormal function is usually leakage of blood through the mitral and tricuspid valves, leading to worsening symptoms, such as shortness of breath.

VHD parameters determined based on the SCG signal(s) (e.g., accelerometer data) when evaluating VHD and PHV dysfunctions can also be used as inputs or weighted inputs to diagnose and/or monitor heart failure and other disease states that may impact cardiac function. For example, VHD parameters can be used in algorithms and systems, including but not limited to, those described in US Patent Publication 2021/0350931, titled METHOD AND SYSTEM FOR HEART CONDITION DETECTION USING AN ACCELEROMETER.

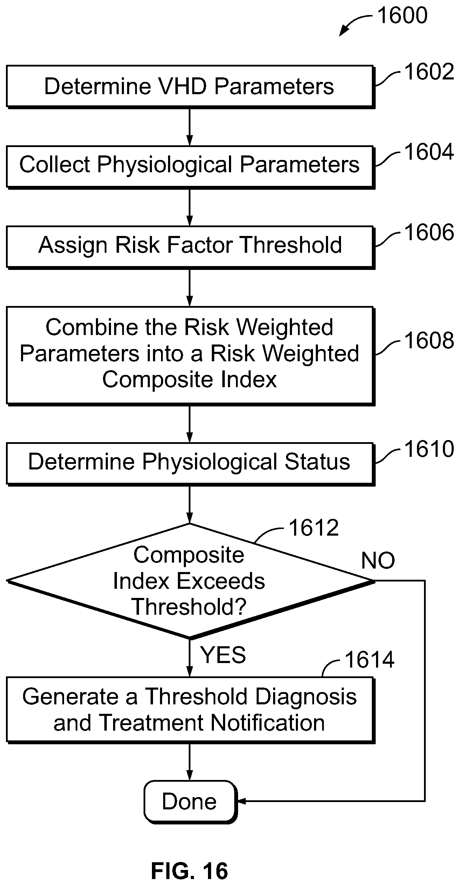

Figure 16 illustrates a computer-implemented method 1600 for using VHD parameters to monitor heart failure and/or other disease states in accordance with embodiments herein. The VHD parameters can be extracted from an accelerometer sensor having one-three axes and used as weighted input(s) to a linear combination or composite of features, along with physiological parameters or parameters extracted using other sensors (e.g., gyroscope), and/or data stored in databases to monitor heart failure or as input to machine learning/AI diagnosis. VHD parameters could also be used to set a threshold for heart failure detection alerts, which if exceeded by physiological parameters or parameters extracted from other sensors would generate alerts. In addition, VHD parameters can be evaluated along with indications of other disease states, such as arterial hypertension, to generate indications of changes in VHD, PHV dysfunction, arterial hypertension, and/or other disease states.

At 1602, the one or more processors determine one or more VHD parameters. For example, referring also to Figs 6, 9, and 10 herein, a VHD parameter can be based on matching scores between the template and SCG segment, based on an alert, number of alerts, lack of alerts over a predetermined period of time, and/or adjustments made to thresholds, counters, pacing, medication, and the like. The VHD parameters can be extracted from an accelerometer, using one to three axes, and/or a gyroscope.

At 1604, optionally, the one or more processors determine physiologic parameters associated with other health related parameters. For example, physiologic data resulting from a six minute walk test or other cardiac stress test, ultrasonic or x-ray evaluation, other implantable electronic devices such as pacemakers, ICDs, CRTDs (e.g., data such as but not limited to percentage of pacing, intracardiac impedances, and the like), medical tests including imaging and blood tests, and/or other patient physiologic parameters such as patient age, weight, height, body mass index (BMI), tobacco use, alcohol use, previously diagnosed health conditions, previous surgeries, etc. The patient physiologic parameters may be collected from a data storage device, including a data storage device of a remote device such as a physician's computing device, government agency computing device, hospital computing device, in home medical device, IMD, ICM, PDE, etc. The physiologic parameters may also be received from monitoring the patient over time, such as by using an accelerometer having one to three axes and/or a gyroscope, and recording and analyzing collected patient data.

At 1606, the one or more processors assign a risk factor threshold for one or more of the VHD parameters and/or one or more of the physiologic parameters to form risk weighted parameters. A risk factor threshold is a weight provided to a risk factor in determining if the pathologic episode is more likely to occur. Risk factors may be given different weights (e.g., values, points, etc.) based on determined criteria. Risk factors can include any health related information that indicates a patient is more likely to experience a pathological episode. Pathologic episodes include heart failure, stroke, syncope, arrythmia, heart attack, asystole, Brady event, neurological episodes, ventricular fibrillation (VF), ventricular tachycardia (VT), a diabetic seizure, and epileptic seizure, or any other type of seizure, episodes that may result from substantial reduction or change in pulmonary arterial pressure and the like. In some cases, none of the VHD parameters and none of the physiologic parameters are assigned a risk factor threshold, while still being included in the overall analysis.

At 1608, the one or more processors combine the risk weighted parameters into a risk weighted composite index. When combining the risk weighted parameters into a risk weighted composite index (e.g. total risk factor, or total risk score) for a patient, each individual risk factor has a risk factor threshold to be considered triggered. In one example, each triggered risk factor can contribute a limited number of points to an overall risk score based on relative strength to other triggers and based on severity of that specific risk factor (e.g., risk factor 1 can contribute 1 point if its threshold is met and up to 2 additional points depending on severity of this risk factor). A combination of risk factors can also contribute additional points to the overall risk score (e.g., risk weighted composite index) with allowed points/severity as before.

The risk weighted composite index represents a general indicator of a degree to which the patient is experiencing a pathologic episode or potential pathologic episode. As a patient's health deteriorates, indicated by one or more characteristics reflected by the risk weighted parameters, the risk weighted composite index will similarly elevate. In one embodiment, a risk model is provided to determine the risk weighted composite index based on VHD parameters and the determined physiologic parameters.

In one embodiment, the one or more processors that receive the VHD parameter data and determined physiologic parameter data may also communicate with a remote database that includes similar patient data of other patients. The other patients' data is utilized to determine the risk weighted composite index and/or resulting treatment diagnosis. In one embodiment, an artificial intelligence (AI) algorithm may be utilized that makes a determination based on variables associated with the remote database to determine a determined pathologic episode, or probability of a determined pathologic episode.

For the AI algorithm, weights may be assigned to differing variables related to the VHD parameter data and determined physiologic parameter data, etc., to make a diagnosis of a pathologic episode such as heart failure. The patient may then undergo an examination to determine if the diagnosis is correct. If the diagnosis is correct, then a reward is provided for the weights providing the correct diagnosis. If the diagnosis is incorrect, no reward is provided, and weights may be varied accordingly.

At 1610, the one or more processors establish a physiologic status of the patient based on the risk weighted composite index. The physiologic status represents the health of the patient. For example, the physiologic status may be that the patient is in good health, or that the health risks to the patient have not changed over an interval. Alternatively, the physiologic status may be that the patient has a high risk of suffering a heart attack, stroke, or other pathologic episode in the near future. The physiologic status may be established by correlating the risk weighted composite index to similarly situated patients. For example, when the risk weighted composite index is a total risk score, a score between 0-5 may be considered a low risk score based on other patients with similar total risk scores that experienced or did not experience a pathologic episode. Meanwhile a score between 5-10 could indicate a moderate health risk, and any score over 10 could represent a high risk score. The low health risk, moderate heath risk, and high health risk are all examples of a physiologic status. Alternatively, a probability of a pathologic episode or other indicator could represent the physiologic status of the patient.

At 1612, the one or more processors make a determination whether the risk weighted composite index exceeds a threshold. In an embodiment when the total risk score is the risk weighted composite index, when a patient scores above a 10 and is within a high risk group, 10 could be considered a threshold score. In this manner, any score 10 or below does not exceed the threshold, while scores above 10 exceed the threshold.

If the risk weighted composite index does not exceed the threshold, flow moves to the right, and no additional action is taken. If the risk weighted composite index does exceed the threshold flow passes to 1614.

At 1614, the one or more processors generate a treatment diagnosis and treatment notification to be provided in connection with the risk weighted composite index. An electronic message (e.g., text, email, voicemail, fax) and/or display on a display can be generated. For example, a medication dose change and/or a office visit may be recommended as a treatment notification. As another example, the one or more processors may generate a diagnosis of a heart failure and/or a particular stage of heart failure, and/or indicate that the patient's level of heart failure has increased. An indication that the patient's VHD has worsened and/or that the patient's prosthetic heart valve should be evaluated can be generated.

In accordance with new and unique aspects herein, the method provides the particular treatment and prophylaxis of monitoring and/or diagnosing heart failure and/or other disease state based on VHD features and other features and information. This provides an improvement in the fields of patient monitoring, as well as improvements to the treatment of heart failure, VHD, and PHV dysfunction.

Embodiments may be implemented in connection with one or more IMDs. Non-limiting examples of IMDs include one or more of neurostimulator devices, implantable leadless monitoring and/or therapy devices, and/or alternative implantable medical devices. For example, the IMD may represent a cardiac monitoring device, pacemaker, cardioverter, cardiac rhythm management device, defibrillator, neurostimulator, leadless monitoring device, leadless pacemaker, and the like. The IMD may measure electrical and/or mechanical information. For example, the IMD may include one or more structural and/or functional aspects of the device(s) described in U.S. Patent Number 9,333,351, entitled NEUROSTIMULATION METHOD AND SYSTEM TO TREAT APNEA, and U.S. Patent Number 9,044,610, entitled SYSTEM AND METHODS FOR PROVIDING A DISTRIBUTED VIRTUAL STIMULATION CATHODE FOR USE WITH AN IMPLANTABLE NEUROSTIMULATION SYSTEM issued June 02, 2015. The IMD may monitor transthoracic impedance, such as implemented by the CorVue algorithm offered by St. Jude Medical. Additionally or alternatively, the IMD may include one or more structural and/or functional aspects of the device(s) described in U.S. Patent Number 9,216,285, entitled LEADLESS IMPLANTABLE MEDICAL DEVICE HAVING REMOVABLE AND FIXED COMPONENTS issued December 22, 2015, and U.S. Patent Number 8,831,747, entitled LEADLESS NEUROSTIMULATION DEVICE AND METHOD INCLUDING THE SAME issued September 09, 2014. Additionally or alternatively, the IMD may include one or more structural and/or functional aspects of the device(s) described in U.S. Patent Number 8,391,980, entitled METHOD AND SYSTEM FOR IDENTIFYING A POTENTIAL LEAD FAILURE IN AN IMPLANTABLE MEDICAL DEVICE issued March 05, 2013, and U.S. Patent Number 9,232,485, entitled SYSTEM AND METHOD FOR SELECTIVELY COMMUNICATING WITH AN IMPLANTABLE MEDICAL DEVICE issued January 05, 2016. Additionally or alternatively, the IMD may be a subcutaneous IMD that includes one or more structural and/or functional aspects of the device(s) described in U.S. Patent Number 10,765,860, entitled SUBCUTANEOUS IMPLANTATION MEDICAL DEVICE WITH MULTIPLE PARASTERNAL-ANTERIOR ELECTRODES filed May 07, 2018; U.S. Patent Number 10,722,704, entitled IMPLANTABLE MEDICAL SYSTEMS AND METHODS INCLUDING PULSE GENERATORS AND LEADS filed May 07, 2018; U.S. Patent Number 11,045,643, entitled SINGLE SITE IMPLANTATION METHODS FOR MEDICAL DEVICES HAVING MULTIPLE LEADS, filed May 07, 2018. Further, one or more combinations of IMDs may be utilized from the above mentioned patents and applications in accordance with embodiments herein. Embodiments may be implemented in connection with one or more subcutaneous implantable medical devices (S-IMDs). For example, the S-IMD may include one or more structural and/or functional aspects of the device(s) described in U.S. Patent Number 10,722,704, entitled IMPLANTABLE MEDICAL SYSTEMS AND METHODS INCLUDING PULSE GENERATORS AND LEADS, filed May 07, 2018; U.S. Patent Number 10,765,860, entitled SUBCUTANEOUS IMPLANTATION MEDICAL DEVICE WITH MULTIPLE PARASTERNAL-ANTERIOR ELECTRODES, filed May 07, 2018. The IMD may represent a passive device that utilizes an external power source and/or an active device that includes an internal power source. The IMD may deliver some type of therapy/treatment, provide mechanical circulatory support and/or merely monitor one or more physiologic characteristics of interest (e.g., PAP, CA signals, impedance, heart sounds). Additionally or alternatively, embodiments may be implemented in connection with one or more passive IMDS (PIMDs). Non-limiting examples of PIMDs may include passive wireless sensors used by themselves or incorporated into or used in conjunction with other IMDs, such as cardiac monitoring devices, pacemakers, cardioverters, cardiac rhythm management devices, defibrillators, neurostimulators, leadless monitoring devices, leadless pacemakers, replacement valves, shunts, grafts, drug elution devices, blood glucose monitoring systems, orthopedic implants, and the like. For example, embodiments may implement one or more structural and/or functional aspects of the device(s) described in U.S. Patent No. 9,265,428 entitled IMPLANTABLE WIRELESS SENSOR, U.S. Patent No. 8,278,941 entitled STRAIN MONITORING SYSTEM AND APPARATUS, U.S. Patent No. 8,026,729 entitled SYSTEM AND APPARATUS FOR IN-VIVO ASSESSMENT OF RELATIVE POSITION OF AN IMPLANT, U.S. Patent No. 8,870,787 entitled VENTRICULAR SHUNT SYSTEM AND METHOD, and U.S. Patent No. 9,653,926 entitled PHYSICAL PROPERTY SENSOR AND ACTIVE ELECTRONIC CIRCUIT AND WIERELESS POWER AND DATA TRANSMISSION.

Additionally or alternatively, embodiments herein may be implemented in connection with the methods and systems described in METHOD AND DEVICE FOR DETECTING RESPIRATION ANOMALY FROM LOW FREQUENCY COMPONENT OF ELECTRICAL CARDIAC ACTIVITY SIGNALS, U.S. Pub. No. 2021/0345891, filed on May 08, 2020.

Additionally or alternatively, embodiments herein may be implemented in connection with the methods and systems described in SYSTEM FOR VERIFYING A PATHOLOGIC EPISODE USING AN ACCELEROMETER, Patent Number 11,980,472, filed March 5, 2021.

Closing Statements

It should be clearly understood that the various arrangements and processes broadly described and illustrated with respect to the Figures, and/or one or more individual components or elements of such arrangements and/or one or more process operations associated of such processes, can be employed independently from or together with one or more other components, elements and/or process operations described and illustrated herein. Accordingly, while various arrangements and processes are broadly contemplated, described, and illustrated herein, it should be understood that they are provided merely in illustrative and non-restrictive fashion, and furthermore can be regarded as but mere examples of possible working environments in which one or more arrangements or processes may function or operate.

As will be appreciated by one skilled in the art, various aspects may be embodied as a system, method, or computer (device) program product. Accordingly, aspects may take the form of an entirely hardware embodiment or an embodiment including hardware and software that may all generally be referred to herein as a "circuit," "module" or "system." Furthermore, aspects may take the form of a computer (device) program product embodied in one or more computer (device) readable storage medium(s) having computer (device) readable program code embodied thereon.

Any combination of one or more non-signal computer (device) readable medium(s) may be utilized. The non-signal medium may be a storage medium. A storage medium may be, for example, an electronic, magnetic, optical, electromagnetic, infrared, or semiconductor system, apparatus, or device, or any suitable combination of the foregoing. More specific examples of a storage medium would include the following: a portable computer diskette, a hard disk, a random-access memory (RAM), a dynamic random-access memory (DRAM), a read-only memory (ROM), an erasable programmable read-only memory (EPROM or Flash memory), a portable compact disc read-only memory (CD-ROM), an optical storage device, a magnetic storage device, or any suitable combination of the foregoing.

Program code for carrying out operations may be written in any combination of one or more programming languages. The program code may execute entirely on a single device, partly on a single device, as a stand-alone software package, partly on single device and partly on another device, or entirely on the other device. In some cases, the devices may be connected through any type of network, including a local area network (LAN) or a wide area network (WAN), or the connection may be made through other devices (for example, through the Internet using an Internet Service Provider) or through a hard wire connection, such as over a USB connection. For example, a server having a first processor, a network interface, and a storage device for storing code may store the program code for carrying out the operations and provide this code through its network interface via a network to a second device having a second processor for execution of the code on the second device.

Aspects are described herein with reference to the Figures, which illustrate example methods, devices, and program products according to various example embodiments. These program instructions may be provided to a processor of a general-purpose computer, special purpose computer, or other programmable data processing device or information handling device to produce a machine, such that the instructions, which execute via a processor of the device implement the functions/acts specified. The program instructions may also be stored in a device readable medium that can direct a device to function in a particular manner, such that the instructions stored in the device readable medium produce an article of manufacture including instructions which implement the function/act specified. The program instructions may also be loaded onto a device to cause a series of operational steps to be performed on the device to produce a device implemented process such that the instructions which execute on the device provide processes for implementing the functions/acts specified.

The units/modules/applications herein may include any processor-based or microprocessor-based system including systems using microcontrollers, reduced instruction set computers (RISC), application specific integrated circuits (ASICs), field-programmable gate arrays (FPGAs), logic circuits, and any other circuit or processor capable of executing the functions described herein. Additionally or alternatively, the modules/controllers herein may represent circuit modules that may be implemented as hardware with associated instructions (for example, software stored on a tangible and non-transitory computer readable storage medium, such as a computer hard drive, ROM, RAM, or the like) that perform the operations described herein. The above examples are exemplary only and are thus not intended to limit in any way the definition and/or meaning of the term "controller." The units/modules/applications herein may execute a set of instructions that are stored in one or more storage elements, to process data. The storage elements may also store data or other information as desired or needed. The storage element may be in the form of an information source or a physical memory element within the modules/controllers herein. The set of instructions may include various commands that instruct the modules/applications herein to perform specific operations such as the methods and processes of the various embodiments of the subject matter described herein. The set of instructions may be in the form of a software program. The software may be in various forms such as system software or application software. Further, the software may be in the form of a collection of separate programs or modules, a program module within a larger program or a portion of a program module. The software also may include modular programming in the form of object-oriented programming. The processing of input data by the processing machine may be in response to user commands, or in response to results of previous processing, or in response to a request made by another processing machine.

It is to be understood that the subject matter described herein is not limited in its application to the details of construction and the arrangement of components set forth in the description herein or illustrated in the drawings hereof. The subject matter described herein is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items.

It is to be understood that the above description is intended to be illustrative, and not restrictive. For example, the above-described embodiments (and/or aspects thereof) may be used in combination with each other. In addition, many modifications may be made to adapt a particular situation or material to the teachings herein without departing from its scope. While the dimensions, types of materials and coatings described herein are intended to define various parameters, they are by no means limiting and are illustrative in nature. Many other embodiments will be apparent to those of skill in the art upon reviewing the above description. The scope of the embodiments should, therefore, be determined with reference to the appended claims, along with the full scope of equivalents to which such claims are entitled. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects or order of execution on their acts.

**Claims**

1. A system (100, 500) for monitoring heart valve operation, comprising:

    memory (260, 522) to store program instructions; and
    one or more processors (220, 520) that, when executing the program instructions, are configured to:

        receive seismocardiograpy (SCG) signals (812) detected by a heart sound sensor (270, 300, 525) along an axis, the SCG signals (812) including heart sound signals for a series of heartbeats over a first time period;
        segment the SCG signals (812) into SCG segments (826) for corresponding heartbeats within the series of heartbeats over the first time period;
        calculate a template based on a first subset of the SCG segments (826);
        compare at least a portion of a second subset of the SCG segments (826) to the template to determine matching scores; and
        generate an alert in response to a select number of the second subset of SCG segments (826) having the matching scores that satisfy a matching threshold.

2. The system of claim 1, wherein the one or more processors (220, 520) are further configured to:

    receive sensed cardiac activity (CA) signals (804) detected by one or more electrodes (114, 126, 512, 526) over the first time period; and
    segment the SCG signals (812) based on peaks (820) detected within the CA signals (804), optionally wherein the CA signals (804) comprise one or more sensed EGM signals or one or more sensed ECG signals.

3. The system of claim 1 or 2, wherein the one or more processors (220, 520) are further configured to calculate the template by averaging the first subset of the SCG segments (826).

4. The system of any one of claims 1 to 3, wherein the one or more processors (220, 520) are further configured to:

   evaluate one or more conditions based on at least one of sleep, activity, and posture; and
   calculate the template in response to at least one of the one or more conditions being satisfied.

5. The system of any one of claims 1 to 4, wherein the one or more processors (220, 520) are further configured to increment a first counter in response to one of the SCG segments (826) not satisfying the matching threshold.

6. The system of claim 5, wherein the one or more processors (220, 520) are further configured to increment a second counter in response to the first counter satisfying a first counter threshold.

7. The system of any one of claims 1 to 6, wherein the one or more processors (220, 520) are further configured to:
   in response to receiving second SCG signals (812) including heart sound signals for a second series of heartbeats over a second time period that is different than the first time period:

   calculate a second template based on a third subset of SCG segments (826) of the second time period;
   compare at least a portion of a fourth subset of SCG segments (826) of the second time period to the second template to determine matching scores; and
   generate an alert in response to the select number of the second subset of SCG segments (826) and the fourth subset of SCG segments (826) having matching scores that satisfy the matching threshold.

8. The system of any one of claims 1 to 7, further comprising an implantable medical device, IMD (100, 500), the IMD (100, 500) comprising:

   one or more electrodes (114, 126, 512, 526) configured to collect cardiac activity (CA) signals (804);
   the heart sound sensor (270, 300, 525); and
   a transceiver (264) configured to wirelessly transmit at least one of the CA signals (804), the SCG signals (812), or the alert to an external device (154).

9. The system of any one of claims 1 to 8, wherein the one or more processors (220, 520) are further configured to:

   receive sensed cardiac activity (CA) signals (804) detected by one or more electrodes (114, 126, 512, 526) over the first time period; and
   analyze at least one of the CA signals (804) and the SCG signals (812) using artificial intelligence.

10. The system of any one of claims 1 to 9, wherein the one or more processors (220, 520) are further configured to reset a counter associated with the select number in response to generating an alert.

11. The system of any one of claims 1 to 10, wherein the matching scores are based on morphology or morphological features of the SCG signals (812) and the template.

12. The system of any one of claims 1 to 11, wherein the one or more processors (220, 520) are further configured to filter the heart sound signals based on one or more frequency ranges or one or more axes associated with the heart sound sensor (270, 300, 525).

13. The system of any one of claims 1 to 12, wherein the one or more processors (220, 520) are further configured to:

   receive second SCG signals (812) detected by the heart sound sensor (270, 300, 525) along a second axis, the second SCG signals (812) including heart sound signals for a series of heartbeats over a second time period;
   segment the second SCG signals (812) into SCG segments (826) for corresponding heartbeats within the series of heartbeats over the second time period;
   calculate a second template based on a first subset of the second SCG segments (826);
   compare at least a portion of a second subset of the second SCG segments (826) to the second template to determine matching scores; and

select the SCG signals (812) or the second SCG signals (812) that have the matching scores that best match the matching threshold.

**14.** The system of any one of claims 1 to 13, wherein the one or more processors (220, 520) adjust, in response to the generation of the alert, a threshold, a counter, a time period, a delay, a parameter to increase or decrease a number of alerts, pacing of an IMD (100, 500), or sensing of an IMD (100, 500).

**15.** A method for monitoring heart valve operation, comprising:

receiving seismocardiograpy (SCG) signals (812) detected by a heart sound sensor (270, 300, 525) along an axis, the SCG signals (812) including heart sound signals for a series of heartbeats over a first time period;

segmenting the SCG signals (812) into SCG segments (826) for corresponding heartbeats within the series of heartbeats over the first time period;

calculating a template based on a first subset of the SCG segments (826);

comparing at least a portion of a second subset of the SCG segments (826) to the template to determine matching scores; and

generating an alert in response to a select number of the second subset of SCG segments (826) having the matching scores that satisfy a matching threshold.

**FIG. 1**

**FIG. 2**

EP 4 699 543 A1

**FIG. 3**

**FIG. 4**

500

510

506

Power
Supply — 516

— 502

Memory — 522

Processor — 520

— 508

Pulse
Generator — 518

Sensing
Circuit — 524

526

Monitoring
Sensor — 525

HR
Sensor — 527

504

514

512

**FIG. 5**

FIG. 6

**FIG. 7**

**FIG. 8**

900

902

Receive SCG signals

904

Segment the SCG signals

906

Calculate template

908

Compare at least a portion of the SCG beats to the template to determine associated matching scores

910

Do SCG segments satisfy Matching Threshold?

YES

NO

912

Generate Alert

914

Stop or evaluate next set of SCG signals

**FIG. 9**

FIG. 10

1100
1102
Obtain SCG Signals at a First Bandwidth and On a First Axis

1104
Determine First Bandwidth First Axis Matching Scores

1106
Second Bandwidth? — YES → Determine Second Bandwidth First Axis Matching Scores  1108

NO

1110
Second Axis? — YES → Determine First Bandwidth Second Axis Matching Scores  1112

NO

1114
Second Bandwidth? — YES → Determine Second Bandwidth Second Axis Matching Scores  1116

NO

1118
Third Axis? — YES → Determine First Bandwidth Third Axis Matching Scores  1120

NO

1122
Second Bandwidth? — YES → Determine Second Bandwidth Third Axis Matching Scores  1124

NO

1126
Select Bandwidth and Axis for Obtaining SCG Signals

FIG. 11

**FIG. 12**

FIG. 13

FIG. 14

**FIG. 15**

FIG. 16

Europäisches
Patentamt

European
Patent Office

Office européen
des brevets

# EUROPEAN SEARCH REPORT

**Application Number**

EP 25 19 4238

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/192888 A1 (THAKUR PRAMODSINGH HIRASINGH [US] ET AL) 7 July 2016 (2016-07-07) * paragraphs [0003], [0023], [0042], [0046], [0047], [0049], [0050], [0053], [0054] * * paragraphs [0062] - [0064], [0070], [0097] * ----- | 1-15 | INV. A61B7/04 |
| X | EP 3 476 299 A1 (ACARIX AS [DK]) 1 May 2019 (2019-05-01) * paragraphs [0031], [0034], [0041] * ----- | 1,15 | |

**TECHNICAL FIELDS SEARCHED (IPC)**

A61B
G16H

The present search report has been drawn up for all claims

| Place of search | Date of completion of the search | Examiner |
|---|---|---|
| Munich | 9 January 2026 | Hemb, Björn |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 25 19 4238

This annex lists the patent family members relating to the patent documents cited in the above-mentioned European search report.
The members are as contained in the European Patent Office EDP file on
The European Patent Office is in no way liable for these particulars which are merely given for the purpose of information.

09-01-2026

| Patent document cited in search report | Publication date | Patent family member(s) | Publication date |
|---|---|---|---|
| US 2016192888 A1 | 07-07-2016 | NONE | |
| EP 3476299 A1 | 01-05-2019 | CN 111263613 A | 09-06-2020 |
| | | EP 3476299 A1 | 01-05-2019 |
| | | EP 3700426 A1 | 02-09-2020 |
| | | ES 2953312 T3 | 10-11-2023 |
| | | JP 7244509 B2 | 22-03-2023 |
| | | JP 2021500182 A | 07-01-2021 |
| | | US 2020367771 A1 | 26-11-2020 |
| | | WO 2019081630 A1 | 02-05-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20210020294 A
- US 6572557 B
- US 6480733 B, Turcott
- US 7272443 B, Min
- US 7308309 B, Koh
- US 6645153 B, Kroll
- US 20220361837 A
- US 20220361818 A
- US 20240424307 A
- US 6937900 B
- US 20210350931 A
- US 20190099087 A
- US 9993167 B
- US 20160007924 A
- US 20230346258 A
- US 20230414951 A
- US 9333351 B
- US 9044610 B
- US 9216285 B
- US 8831747 B
- US 8391980 B
- US 9232485 B
- US 10765860 B
- US 10722704 B
- US 11045643 B
- US 9265428 B
- US 8278941 B
- US 8026729 B
- US 8870787 B
- US 9653926 B
- US 20210345891 A
- US 11980472 B

### Non-patent literature cited in the description

- **C. YANG et al.** An Open-Access Database for the Evaluation of Cardio-Mechanical Signals From Patients with Valvular Heart Diseases. *Front. Physiol.*, 2021, vol. 12 (750221), https://doi.org/10.3389/fphys.2021.750221